(19) Europäisches Patentamt
European Patent Office
Office européen des brevets

(11) EP 1 731 548 B1

(12) FASCICULE DE BREVET EUROPEEN

(45) Date de publication et mention de la délivrance du brevet:
**11.03.2009 Bulletin 2009/11**

(51) Int Cl.:
***C08G 77/442** (2006.01)* ***G01N 21/64** (2006.01)*
***G01N 21/77** (2006.01)* ***C08F 283/12** (2006.01)*
***G01N 33/22** (2006.01)*

(21) Numéro de dépôt: **06115179.1**

(22) Date de dépôt: **08.06.2006**

(54) **Utilisation de polysiloxanes fluorescents dans des capteurs chimiques pour la detection ou le dosage de composes nitres**

Verwendung von fluoreszierenden Polysiloxanen in chemischen Sensoren zum Nachweis oder zur Bestimmung von Nitroverbindungen

Use of fluorescent polysiloxanes in chemical sensors for detection or dosage of nitrated compounds

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **09.06.2005 FR 0551556**

(43) Date de publication de la demande:
**13.12.2006 Bulletin 2006/50**

(73) Titulaire: **COMMISSARIAT A L'ENERGIE ATOMIQUE**
**75015 Paris (FR)**

(72) Inventeurs:
- **Hairault, Lionel**
  **37150 Blere (FR)**
- **Pasquinet, Eric**
  **37550, Saint Avertin (FR)**
- **Montmeat, Pierre**
  **37520, La Riche (FR)**
- **Boulhana, Ahmed**
  **Laboratoire de Chimie Organique**
  **FES (MA)**
- **Guida-Pietrasanta, Francine**
  **34090, Montpellier (FR)**
- **Boutevin, Bernard**
  **34090, Montpellier (FR)**

(74) Mandataire: **Poulin, Gérard et al**
**Société BREVATOME**
**3, rue du Docteur Lancereaux**
**75008 Paris (FR)**

(56) Documents cités:
**WO-A-00/42418**
**WO-A-98/22795**
**US-A- 5 107 008**
**US-A- 5 409 666**
**US-A- 5 700 897**

- **MCGILL R A ET AL: "The design of functionalized silicone polymers for chemical sensor detection of nitroaromatic compounds" SENSORS AND ACTUATORS B, ELSEVIER SEQUOIA S.A., LAUSANNE, CH, vol. 65, no. 1-3, 30 juin 2000 (2000-06-30), pages 5-9, XP004208582 ISSN: 0925-4005**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

### DOMAINE TECHNIQUE

La présente invention se rapporte à l'utilisation de polysiloxanes fluorescents en tant que matériaux sensibles dans des capteurs destinés à détecter ou à doser des composés nitrés, et en particulier des composés nitroaromatiques tels que le nitrobenzène (NB), le dinitrobenzène (DNB), le trinitrobenzène (TNB), le nitrotoluène (NT), le 2,4-dinitrotoluène (DNT), le 2,4,6-trinitrotoluène (TNT) et analogues.

Elle se rapporte également à des polysiloxanes fluorescents, à un procédé pour les préparer ainsi qu'à des capteurs chimiques les comprenant en tant que matériaux sensibles.

L'invention trouve notamment application dans la détection d'explosifs, que ce soit en vue d'assurer la sécurité de lieux publics comme les aéroports, de contrôler la licéité de marchandises en circulation sur un territoire, de lutter contre le terrorisme, de procéder à des opérations de désarmement, de localiser des mines antipersonnel ou encore de dépolluer des sites industriels ou militaires.

Elle trouve également application dans la protection de l'environnement, en particulier pour le contrôle et la surveillance de la pollution atmosphérique et de la qualité d'ambiances plus ou moins confinées, ainsi que dans la surveillance à des fins sécuritaires, de sites industriels fabriquant, stockant et/ou manipulant des composés nitrés.

### ÉTAT DE LA TECHNIQUE ANTÉRIEURE

La détection d'explosifs est un problème d'intérêt crucial, notamment en matière de sécurité civile.

A l'heure actuelle, plusieurs méthodes sont utilisées pour détecter des vapeurs de composés nitrés entrant dans la constitution des explosifs, comme l'emploi de chiens "*renifleurs*" dressés et entraînés à cet effet, l'analyse en laboratoire, par exemple par chromatographie couplée à un spectromètre de masse ou à un détecteur à capture d'électrons, d'échantillons prélevés sur site, ou encore la détection infrarouge.

Ces méthodes font, d'une manière générale, preuve d'une grande sensibilité, ce qui est primordial en matière de détection d'explosifs compte tenu de la très faible concentration en vapeurs de composés nitrés qui règne au voisinage d'un explosif. Elles ne donnent toutefois pas totalement satisfaction.

Ainsi, l'utilisation de chiens *"renifleurs"* présente l'inconvénient de nécessiter une longue formation des chiens et de leurs maîtres et d'être inadaptée à des opérations prolongées en raison de ce que la durée d'attention des chiens est limitée.

Quant aux autres méthodes, l'encombrement des appareillages qu'elles utilisent, leur consommation d'énergie et leurs coûts de mise en oeuvre s'opposent au développement de systèmes de détection aisément transportables et autonomes et, partant, aptes à être utilisés sur tout type de sites.

Depuis quelques années, le développement de capteurs capables de détecter en temps réel des espèces chimiques gazeuses est en plein essor. Le fonctionnement de ces capteurs est basé sur l'utilisation d'un film d'un matériau sensible, c'est-à-dire d'un matériau dont au moins une propriété physique est modifiée au contact des molécules gazeuses recherchées, qui revêt un système de transduction qui mesure en temps réel toute variation de cette propriété physique et permet de mettre ainsi en évidence la présence des molécules gazeuses recherchées.

Les avantages des capteurs chimiques par rapport aux méthodes précitées sont multiples : instantanéité des résultats, possibilité de miniaturisation et, donc, portabilité, maniabilité et autonomie importante, faibles coûts de fabrication et d'exploitation, etc.

Toutefois, il est évident que leurs performances sont extrêmement variables selon la nature du matériau sensible utilisé.

A ce jour, un certain nombre de travaux visant à rechercher des matériaux sensibles pour la détection de composés nitrés, et plus particulièrement de composés nitroaromatiques, a été réalisé.

A ainsi été étudiée la possibilité d'utiliser des polyéthylène glycols, du silicium poreux, des adsorbants du type charbon végétal, des composés organiques cycliques (phtalocyanine de cuivre, cyclodextrines, cavitands), des dendrimères, des amines et des polysiloxanes.

Concernant ces derniers, ont été proposés des polysiloxanes fonctionnalisés par un groupe hexafluoroisopropanol (McGill et *al., Sensors and Actuators B65,* 5-9, 2000 **[1]**) et des polysiloxanes liés de façon covalente à un dérivé de cyclodextrine (WO-A-98/22795, **[2]**).

Ces polysiloxanes étant dénués de groupe fluorophore, ils ne conviennent pas à la réalisation de capteurs optiques dits *"à fluorescence",* c'est-à-dire dont le fonctionnement est basé sur une transduction par variation de fluorescence, ce qui limite considérablement leur utilisation.

Les Inventeurs se sont donc fixé pour but de trouver des composés qui soient aptes à servir de matériaux sensibles dans des capteurs destinés à détecter ou doser des composés nitrés, et en particulier dans des capteurs utilisant une transduction par variation de fluorescence.

## EXPOSÉ DE L'INVENTION

Ce but et d'autres encore sont atteints par la présente invention qui a, en premier lieu, pour objet l'utilisation d'au moins un polysiloxane fluorescent comprenant un motif répétitif siloxane répondant à l'une des formules générales (I) et (II) ci-après :

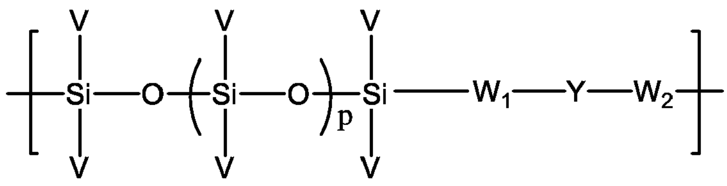

(I)

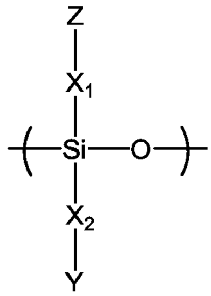

(II)

dans lesquelles :

$W_1$ et $W_2$, qui peuvent être identiques ou différents, représentent un groupe hydrocarboné comprenant de 2 à 50 atomes de carbone et comportant éventuellement un ou plusieurs hétéroatomes et/ou une ou plusieurs fonctions chimiques comprenant au moins un hétéroatome ;
V représente un atome d'hydrogène ou un groupe hydrocarboné comprenant de 1 à 50 atomes de carbone et comportant éventuellement un ou plusieurs hétéroatomes et/ou une ou plusieurs fonctions chimiques comprenant au moins un hétéroatome ;
p est un nombre entier allant de 0 à 10 000 ;
Y représente un groupe fluorophore ;
$X_1$ et $X_2$, qui peuvent être identiques ou différents, représentent une liaison simple ou un groupe hydrocarboné, comprenant de 1 à 50 atomes de carbone et comportant éventuellement un ou plusieurs hétéroatomes et/ou une ou plusieurs fonctions chimiques comprenant au moins un hétéroatome ; et
Z représente un atome d'hydrogène ou a la même signification que Y ; en tant que matériau sensible dans un capteur chimique pour la détection ou le dosage d'au moins un composé nitré.

Conformément à l'invention, chacun des groupes hydrocarbonés susceptibles d'être présents dans la formule générale (I) - en tant que $W_1$, $W_2$ et V - et dans la formule générale (II) — en tant que $X_1$ et $X_2$ - peut être un groupe saturé, insaturé, aliphatique (linéaire ou ramifié), cyclique dès lors qu'il comporte 3 ou plus de 3 atomes de carbone, ou bien encore partiellement aliphatique et partiellement cyclique dès lors qu'il comporte 4 ou plus de 4 atomes de carbone. De plus, dans le cas où ces groupes hydrocarbonés comprennent, ou sont constitués par, un ou plusieurs cycles insaturés, alors ce ou ces cycles peuvent être des cycles aromatiques (ou hétéroaromatiques s'ils comportent un ou plusieurs hétéroatomes) ou bien des cycles non aromatiques comme, par exemple, des cycles éthyléniques ou acétyléniques.

Par ailleurs, lorsque les groupes hydrocarbonés susceptibles d'être présents dans la formule générale (I) - en tant que $W_1$, $W_2$ et V - et dans la formule générale (II) - en tant que $X_1$ et $X_2$ - comportent un ou plusieurs hétéroatomes et/ou une ou plusieurs fonctions chimiques comprenant au moins un hétéroatome, alors cet ou ces hétéroatomes et/ou

cette ou ces fonctions chimiques peuvent être portés latéralement par ces groupes ou former pont dans ces groupes dès lors que ces derniers comprennent 2 ou plus de 2 atomes de carbone. Dans le cas des groupes hydrocarbonés représentés par V, cet hétéroatome ou l'un de ces hétéroatomes ou cette fonction chimique ou l'une de ces fonctions chimiques peut, en outre, se situer à l'extrémité de ces groupes.

Conformément à l'invention, tous ces groupes hydrocarbonés peuvent comporter jusqu'à 50 atomes de carbone. On préfère néanmoins qu'ils ne comportent pas plus de 30 atomes de carbone et, mieux encore, pas plus de 15 atomes de carbone.

Dans ce qui précède et ce qui suit, on entend par "*hétéroatome*", un atome autre que de carbone ou d'hydrogène comme, par exemple, un atome d'oxygène, d'azote, de soufre, d'halogène, de phosphore, de bore ou encore de silicium, les atomes d'oxygène, d'azote, de soufre et d'halogène étant préférés.

On entend par *"fonction chimique comprenant au moins un hétéroatome",* une fonction chimique comportant un ou plusieurs atomes autres que de carbone ou d'hydrogène et, notamment, une fonction comportant un ou plusieurs atomes d'oxygène, d'azote, de soufre et/ou d'halogène.

Cette fonction chimique peut, en particulier, être choisie parmi les fonctions -COOH, $-COOR^1$, -CHO, -CO-, -OH, $-OR^1$, -SH, $-SR^1$, $-SO_2R^1$, $-NH_2$, $-NHR^1$, $-NR^1R^2$, $-CONH_2$, $-CONHR^1$, $-CONR^1R^2$, $-C(Hal)_3$, $-OC(Hal)_3$, -C(O)Hal, -CN, -COOCHO, $-COOCOR^1$ et phénol, dans lesquelles :

- $R^1$ représente un groupe hydrocarboné, linéaire, ramifié ou cyclique, saturé ou insaturé, comprenant de 1 à 100 atomes de carbone et, de préférence, de 1 à 30 atomes de carbone, ou une liaison covalente dans le cas où ladite fonction chimique forme pont dans un groupe hydrocarboné ;
- $R^2$ représente un groupe hydrocarboné, linéaire, ramifié ou cyclique, saturé ou insaturé, comprenant de 1 à 100 atomes de carbone et, de préférence, de 1 à 30 atomes de carbone, ce groupe pouvant être identique ou différent du groupe hydrocarboné représenté par $R^1$ ; tandis que
- Hal représente un atome d'halogène, par exemple un atome de fluor, de chlore ou de brome.

On entend par *"cycle aromatique",* un système cyclique satisfaisant à la règle de Hückel, c'est-à-dire présentant un nombre d'électrons π délocalisés égal à (4n + 2), et par *"cycle hétéro-aromatique",* un cycle aromatique tel qu'il vient d'être défini mais comprenant un ou plusieurs hétéroatomes. A titre d'exemples de cycles aromatiques susceptibles d'être utilisés, on peut citer le cyclopentadiényle, le phényle, le benzyle, le biphényle, le phénylacétylényle, le pyrényle ou l'anthracényle, tandis qu'à titre d'exemples de cycles hétéroaromatiques, on peut citer le furannyle, le pyrrolyle, le thiophényle, l'oxazolyle, le pyrazolyle, le thiazolyle, l'imidazolyle, le triazolyle, le pyridinyle, le pyranyle, le quinoléinyle, le pyrazinyle et le pyrimidinyle.

Par ailleurs, on entend par *"groupe fluorophore",* un groupe capable d'émettre une lumière fluorescente en réponse à une excitation lumineuse appropriée.

Un tel groupe peut notamment être un groupe naphtacényle, naphtalényle, acénaphtyle, dansyle, xanthanyle, thioxanthanyle, anthraquinyle, acridinyle, rhodaminyle, fluorényle, fluoranthrényle, pentacényle, tétrahydrochrysényle, carbazolyle, pyridyle, imidazolyle, phénothiazolyle, toluidinyle, 1H-benzimidazo-[2,1-a]benz[de]isoquinoléinyle, 4-(2'-phényl)vinylphényle, anthracényle, pyrényle, pérylényle, benzopérylényle, benzo[k]fluoranthényle, benzothiazolyle, benzimidazolyle, oligo(phénylènevinylidényle), oligo-(phénylèneéthynylényle), fluorescéinyle, coumarinyle, pyridyloxazolyle, benzoxazolyle, benzoxadiazolyle, 5,5'-bis(4-aminophényl)-2,2'-bifuryle ou un dérivé fonctionnel de ceux-ci.

Avantageusement, le groupe fluorophore est un groupe pyrényle.

Conformément à l'invention, on préfère, parmi les motifs répétitifs de formule générale (I), ceux dans lesquels $W_1$ et $W_2$ représentent, indépendamment l'un de l'autre, un groupe alkylène comprenant de 2 à 10 atomes de carbone, V est un groupe alkyle comprenant de 1 à 10 atomes de carbone, tandis que p et Y sont tels que précédemment définis, et, parmi ceux-ci, ceux dans lesquels $W_1$ et $W_2$ sont tous les deux un groupe propylène, V est un groupe méthyle et p vaut 1.

Ainsi, par exemple, des polysiloxanes comprenant comme motif répétitif, le motif de formule (III) ci-après :

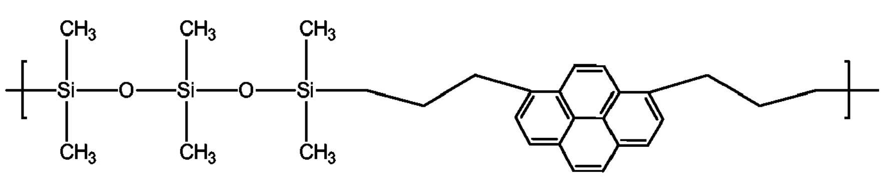

(III)

se sont révélés représenter des matériaux sensibles particulièrement intéressants.

Par ailleurs, parmi les motifs répétitifs de formule générale (II), on préfère ceux dans lesquels $X_1$ représente

un groupe alkylène comprenant de 1 à 10 atomes de carbone, $X_2$ représente un groupe alkylène comprenant de 2 à 10 atomes de carbone, Z est un atome d'hydrogène tandis que Y est tel que précédemment défini, et, parmi ceux-ci, ceux dans lesquels $X_1$ est un groupe méthylène et $X_2$ est un groupe propylène.

Un tel motif répétitif est, par exemple, le motif répétitif de formule (IV) ci-après :

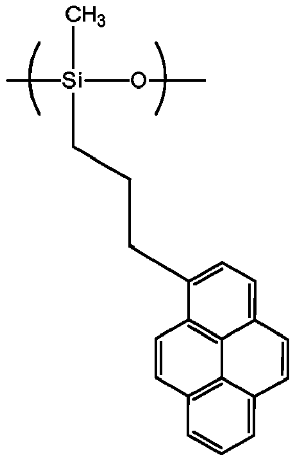

(IV)

Conformément à l'invention, le polysiloxane peut être un homopolymère, c'est-à-dire qu'il peut n'être constitué que par un seul motif répétitif de formule générale (I) ou (II), ce motif pouvant être répété jusqu'à 10 000 fois.

Des exemples de tels homopolymères sont, par exemple, des homopolymères constitués du motif répétitif de formule (III) ou du motif répétitif de formule (IV).

En variante, le polysiloxane peut également être un copolymère, auquel cas il peut aussi bien être constitué de plusieurs motifs répétitifs siloxanes différents mais répondant tous à l'une des formules générales (I) et (II), que comprendre un ou plusieurs motifs répétitifs siloxanes répondant à l'une de ces formules et un ou plusieurs motifs répétitifs siloxanes autres.

Ainsi, le polysiloxane peut notamment être un copolymère constitué d'un premier motif répétitif siloxane répondant à l'une des formules générales (I) et (II) dans lesquelles $W_1$, $W_2$, V, p, $X_1$, $X_2$ et Z sont tels que précédemment définis, et d'un deuxième motif siloxane répondant à la formule générale (V) ci-après :

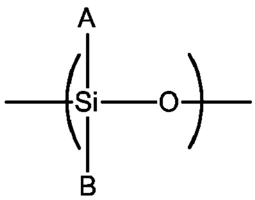

(V)

dans laquelle A et B, qui peuvent être identiques ou différents, représentent un atome d'hydrogène ou un groupe hydrocarboné comprenant de 1 à 50 atomes de carbone et comportant éventuellement un ou plusieurs hétéroatomes et/ou une ou plusieurs fonctions chimiques comprenant au moins un hétéroatome.

Là également, lorsque A et/ou B représentent, dans la formule générale (V), un groupe hydrocarboné, alors ce groupe peut être saturé, insaturé, aliphatique (linéaire ou ramifié), cyclique dès lors qu'il comporte 3 ou plus de 3 atomes de carbone, ou bien encore partiellement aliphatique et partiellement cyclique dès lors qu'il comporte 4 ou plus de 4 atomes de carbone. Si ce groupe hydrocarboné comprend, ou est constitué par, un ou plusieurs cycles insaturés, ce ou ces cycles peuvent être des cycles aromatiques (ou hétéroaromatiques s'ils comportent un ou plusieurs hétéroatomes) ou bien des cycles non aromatiques, tandis que, si ce groupe hydrocarboné comporte un ou plusieurs hétéroatomes et/ou une ou plusieurs fonctions chimiques comprenant un hétéroatome, cet ou ces hétéroatomes et cette ou ces fonctions chimiques peuvent être portés latéralement par ce groupe, former pont dans ce groupe dès lors qu'il comprend 2 ou plus de 2 atomes de carbone, un hétéroatome ou une fonction chimique ou l'une de ces fonctions

chimiques pouvant aussi se situer à son extrémité.

De préférence, le deuxième motif siloxane est un motif diméthylsiloxane (A = B = $CH_3$).

Des exemples de tels copolymères sont notamment des copolymères constitués du motif répétitif de formule (IV) et du motif répétitif diméthylsiloxane.

Conformément à l'invention, ces copolymères peuvent être aléatoires, alternés ou séquencés, mais ils sont préférentiellement aléatoires.

Qu'il s'agisse d'un homopolymère ou d'un copolymère, la masse moléculaire du polysiloxane en masse est généralement supérieure ou égale à 400 g/mole, et va, de préférence, de 400 à 10 000 g/mole et, mieux encore, de 400 à 2 000 g/mole.

De préférence, le polysiloxane est présent dans le capteur sous la forme d'un film mince qui recouvre l'une ou les deux faces d'un substrat.

Toutefois, il peut également être présent sous la forme d'un monolithe comme, par exemple, un cylindre présentant une certaine porosité de sorte à rendre accessible aux composés nitrés destinés à être détectés ou dosés l'ensemble des motifs formant ledit polysiloxane.

Lorsqu'il se présente sous la forme d'un film mince, ce dernier a, de préférence, une épaisseur allant de 10 angströms à 100 microns.

Un tel film peut être obtenu par l'une quelconque des techniques proposées à ce jour pour réaliser un film mince sur la surface d'un substrat, par exemple :

* par pulvérisation, par dépôt à la tournette (*"spin coating"*) ou par dépôt à la goutte (*"drop coating"*) sur le substrat d'une solution du polysiloxane dans un solvant organique ;
* par trempage-retrait ("*dip coating*") du substrat dans une solution du polysiloxane dans un solvant organique ; ou encore
* par la technique de Langmuir-Blodgett.

Le substrat ainsi que le système de mesure du capteur sont choisis en fonction de la propriété physique du polysiloxane dont les variations induites par la présence de composés nitrés sont destinées à être mesurées par le capteur.

En l'espèce, on préfère mesurer les variations de l'intensité de la fluorescence émise par le polysiloxane, compte tenu de la sensibilité qu'offre ce type de transduction.

Aussi, le capteur est-il, de préférence, un capteur optique conçu pour mesurer les variations de l'intensité de la fluorescence émise par le polysiloxane en présence d'un composé nitré.

Le principe de fonctionnement des capteurs optiques à fluorescence a notamment été décrit par B. Valeur dans Molecular Fluorescence : Principles and Applications, 2002, Ed. WILEY VCH, New York **[3].**

Ces capteurs comprennent généralement un substrat en verre de qualité optique dont l'une des faces est recouverte d'un film mince du matériau sensible. L'intensité de la fluorescence émise par le matériau sensible peut être mesurée sur l'ensemble du spectre d'émission de ce matériau. Toutefois, il est préférable d'effectuer les mesures d'intensité de fluorescence à la longueur d'onde d'émission donnant les valeurs d'intensité maximales pour la longueur d'onde d'excitation conduisant, elle, au meilleur rapport signal/bruit pour l'acquisition des intensités de fluorescence.

Il est également possible d'utiliser un polysiloxane tel que précédemment défini comme matériau sensible dans des capteurs conçus pour mesurer les variations d'une propriété physique autre que l'intensité de fluorescence. Ainsi, notamment, les polysiloxanes de formules générales (I) et (II) sont tout à fait aptes à être utilisés dans des capteurs gravimétriques dont le fonctionnement est basé sur la mesure des variations de la masse du matériau sensible.

A titre d'exemples de capteurs gravimétriques, on peut citer les capteurs du type à microbalance à quartz, les capteurs à ondes de surface, plus connus sous l'acronyme anglo-saxon *SAW* ("*Surface Acoustic Wave*"), tels que les capteurs à ondes de Love et les capteurs à ondes de Lamb, ainsi que les microleviers.

Parmi les capteurs gravimétriques, on préfère plus particulièrement les capteurs du type microbalance à quartz. Ce type de capteurs, dont le principe de fonctionnement a été décrit par J.A.O. Sanchez-Pedrono et al. dans Anal. Chem. Acta, 182, 1986, 285 **[4],** comprend, schématiquement, un substrat piézo-électrique (ou résonateur), généralement un cristal de quartz recouvert sur ses deux faces d'une couche métallique, par exemple d'or ou de platine, servant d'électrode. Le matériau sensible recouvrant l'une ou les deux faces du substrat, toute variation de masse de ce matériau se traduit par une variation de la fréquence de vibration du substrat.

Conformément à l'invention, le capteur peut être de type *"multicapteur",* c'est-à-dire qu'il peut être constitué de plusieurs capteurs élémentaires qui comprennent des matériaux sensibles différents les uns des autres, ou qui sont munis de substrats et de systèmes de mesure différents les uns des autres comme, par exemple, un ou plusieurs capteurs à base de fluorescence et/ou un ou plusieurs capteurs gravimétriques, l'essentiel étant que l'un au moins de ces capteurs élémentaires comprenne au moins un polysiloxane tel que précédemment défini, en tant que matériau sensible.

Conformément à l'invention, le composé nitré destiné à être détecté ou dosé est, de préférence, choisi parmi les composés nitroaromatiques, les nitramines, les nitrosamines et les esters nitriques, ce composé pouvant aussi bien se présenter sous forme solide ou liquide que sous forme de vapeurs, la forme vapeurs étant toutefois préférée.

A titre d'exemples de composés nitroaromatiques, on peut citer le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'aminodinitrotoluène, le dinitrotrifluorométhylbenzène, le chlorodinitrotrifluorométhylbenzène, l'hexanitrostilbène ou encore le trinitrophénol (ou acide picrique).

Les nitramines sont, elles, par exemple la cyclotétraméthylènetétranitramine (ou octogène), la cyclotriméthylènetrinitramine (ou hexogène) et la trinitrophénylméthylnitramine (ou tétryle), tandis que les nitrosamines sont, par exemple, la nitrosodiméthylamine.

Quant aux esters nitriques, il s'agit, par exemple, de pentrite, de dinitrate d'éthylène glycol, de dinitrate de diéthylène glycol, de nitroglycérine ou de nitroguanidine.

Des capteurs comprenant un polysiloxane fluorescent tel que précédemment défini en tant que matériau sensible, se sont révélés présenter de nombreux avantages, et notamment :

* une aptitude à détecter les composés nitrés, et en particulier les composés nitroaromatiques, avec une très grande sensibilité - puisqu'ils sont capables de détecter leur présence à des concentrations de l'ordre du ppm (partie par million), voire du dixième de ppm - doublée d'une spécificité vis-à-vis de ces composés ;
* une rapidité de réponse et une reproductibilité de cette réponse ;
* une stabilité des performances dans le temps et une durée de vie très satisfaisante ;
* une aptitude à fonctionner en continu ;
* un coût de fabrication compatible avec une production de capteurs en série, une très faible quantité de polysiloxane (c'est-à-dire, en pratique de quelques mg) étant nécessaire pour la fabrication d'un capteur, et
* la possibilité d'être miniaturisés et, ainsi, d'être aisément transportables et manipulables sur tout type de sites.

Ils sont donc particulièrement utiles pour détecter des explosifs, notamment dans des lieux publics.

A la connaissance des Inventeurs, des polysiloxanes fluorescents ayant pour caractéristique que les groupes fluorophores, qui sont à la base de leur fluorescence, sont incorporés dans la chaîne polysiloxane n'ont jamais été décrits dans l'état de la technique et sont donc nouveaux.

Aussi, l'invention a-t-elle, également, pour objet un polysiloxane fluorescent qui comprend un motif répétitif siloxane répondant à la formule générale (I) telle que précédemment définie, ainsi qu'un capteur chimique comprenant au moins ce polysiloxane fluorescent en tant que matériau sensible.

Les caractéristiques de ce polysiloxane et du capteur chimique le comprenant sont analogues à celles précédemment énoncées dans le cadre de l'utilisation d'un polysiloxane comprenant un motif répétitif de formule générale (I) dans un capteur chimique pour la détection ou le dosage d'au moins un composé nitré.

D'une manière générale, les polysiloxanes (homopolymères) constitués d'un motif répétitif siloxane de formule générale (I) peuvent être obtenus par polycondensation d'un composé de formule (VI) ci-après :

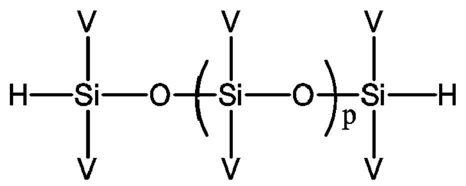

(VI)

dans laquelle V et p ont les mêmes significations que dans la formule générale (I), avec un composé de formule (VII) ci-après :

$P_1—Y—P_2$ (VII)

dans laquelle :

Y a la même signification que dans la formule générale (II) ; et
$P_1$ et $P_2$, qui peuvent être identiques ou différents, représentent un groupe hydrocarboné qui comprend de 2 à 50

atomes de carbone et qui est terminé par un groupe $-CH=CH_2$.

Les polysiloxanes (copolymères) constitués de plusieurs motifs répétitifs siloxane différents, dont l'un au moins est un motif répétitif de formule générale (I), peuvent être obtenus par polycondensation d'un monomère de formule (VIII) ci-après :

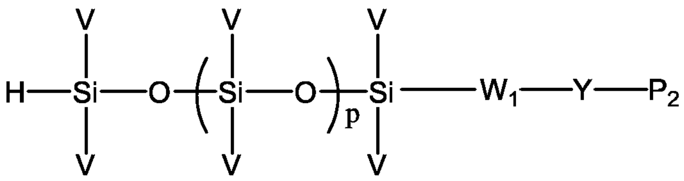

(VIII)

dans laquelle :

$W_1$, V, Y, et p ont les mêmes significations que dans la formule générale (I) ; et

$P_2$ représente un groupe hydrocarboné qui comprend de 2 à 50 atomes de carbone et qui est terminé par un groupe $-CH=CH_2$ ;

ou d'un oligomère résultant de la condensation de plusieurs monomères de formule (VIII), avec au moins un autre monomère siloxane ou au moins un autre oligomère formé à partir de ce dernier monomère.

Ces condensations sont avantageusement réalisées en présence d'un catalyseur, lequel peut être n'importe quel catalyseur classiquement employé pour effectuer des hydrosilylations comme, par exemple, un catalyseur à base de métaux de transition tels que le platine, le rhodium ou le ruthénium, ou bien un peroxyde.

Les polysiloxanes comprenant un motif répétitif de formule générale (II) peuvent, quant à eux, être obtenus en greffant un groupe fluorophore sur l'ensemble ou sur certains des motifs répétitifs siloxane d'un polysiloxane existant, ou en polycondensant des monomères siloxanes porteurs d'un groupe fluorophore et munis de groupes fonctionnels réactifs entre eux ou avec un polysiloxane existant dont les extrémités sont munies de groupes fonctionnels réactifs.

D'autres caractéristiques et avantages de l'invention apparaîtront mieux à la lecture du complément de description qui suit, qui se rapporte à des exemples de synthèse de polysiloxanes fluorescents aptes à servir de matériaux sensibles dans des capteurs chimiques pour la détection ou le dosage de composés nitrés ainsi qu'à des exemples d'utilisation de ces polysiloxanes fluorescents comme matériaux sensibles dans des capteurs chimiques et de démonstration de leurs propriétés.

Bien entendu, ces exemples ne sont donnés qu'à titre d'illustration de l'objet de l'invention et ne constituent en aucun cas une limitation de cet objet.

## BRÈVE DESCRIPTION DES DESSINS

La figure 1 représente l'évolution de l'intensité de la fluorescence émise par un capteur optique à fluorescence comprenant un film mince d'un polysiloxane fluorescent utile selon l'invention, lorsque ce capteur est exposé successivement à de l'air et à des vapeurs de 2,4-dinitrotrifluorométhoxybenzène (DNTFMB).

La figure 2 représente l'évolution de l'intensité de la fluorescence émise par un capteur optique à fluorescence comprenant un film mince d'un polysiloxane fluorescent utile selon l'invention, lorsque ce capteur est exposé successivement à de l'air et à des vapeurs de DNTFMB, dichlorométhane, cyclohexane, méthyléthylcétone, toluène, méthylisobutylcétone et acétate d'éthyle.

La figure 3 représente, sous la forme d'histogrammes, la sensibilité vis-à-vis du DNTFMB, pour une durée d'exposition de 10 minutes, de trois capteurs optiques à fluorescence comprenant pour le premier, un film mince de pyrène, et pour les deux autres, un film mince de polysiloxanes fluorescents utiles selon l'invention.

La figure 4 représente l'évolution de l'intensité de la fluorescence émise par un capteur optique à fluorescence comprenant un film mince d'un polysiloxane fluorescent utile selon l'invention, lorsque ce capteur est exposé successivement à de l'air et à des vapeurs de DNTFMB.

La figure 5 représente l'évolution de l'intensité de la fluorescence émise par un capteur optique à fluorescence comprenant un film mince d'un polysiloxane fluorescent utile selon l'invention, lorsque ce capteur est exposé successivement à de l'air et à des vapeurs de 2,4-dinitrotoluène (DNT) et de dinitrobenzène (DNB).

La figure 6 représente les variations de fréquence de vibration du quartz d'un capteur gravimétrique à microbalance à quartz comprenant un film mince d'un polysiloxane fluorescent utile selon l'invention, lorsque ce capteur est exposé successivement à de l'air et à des vapeurs de DNTFMB.

## EXPOSÉ DÉTAILLÉ DE MODES DE RÉALISATION PARTICULIERS

Dans les exemples 3 à 7 qui suivent, les mesures d'intensité de fluorescence sont réalisées au moyen d'un fluorimètre FluoroMax-3 de la société JOBIN YVON, en régime dynamique dans une cellule balayée à 20 L/h et thermostatée à 25°C.

Ces mesures sont effectuées en utilisant la longueur d'onde d'excitation du matériau sensible utilisé conduisant au meilleur rapport signal/bruit pour l'acquisition des intensités de fluorescence, et à la longueur d'onde d'émission donnant les intensités de fluorescence maximales pour cette longueur d'onde d'excitation. Les longueurs d'onde d'émission et d'excitation ainsi retenues sont précisées dans chaque exemple.

### Exemple 1 : Synthèse de polysiloxanes fluorescents comprenant un motif répétitif de formule générale (II)

On synthétise trois polysiloxanes fluorescents, respectivement désignés ci-après polysiloxane A, B et C, et correspondant :

- pour le premier, à un copolymère aléatoire constitué du motif répétitif de formule (IV) ci-avant et du motif répétitif diméthylsiloxane dans un rapport de 0,04 motif de formule (IV) pour 0,96 motif diméthylsiloxane ;
- pour le second, à un copolymère aléatoire constitué du motif répétitif de formule (IV) ci-avant et du motif répétitif diméthylsiloxane dans un rapport de 0,47 motif de formule (IV) pour 0,53 motif diméthylsiloxane ; et
- pour le troisième, à un homopolymère à motif répétitif de formule (IV) ci-avant ; en utilisant les protocoles opératoires suivants :

Polysiloxane A :

Dans un bicol de 25 mL sous argon, on introduit 0,1 g d'allylpyrène préalablement synthétisé comme décrit par Takuwa et al. (J. Chem. Soc. Perkin Trans 1, 1309, 1998). On porte à 110°C et on injecte 5 μL de catalyseur de Karstedt (Pt/1,3-divinyl-1,1,3,3-tétraméthyldisiloxane en solution dans le xylène, disponible chez ABCR sous la référence SIP6831-0). Après 15 minutes, on introduit 1 g de polydiméthylméthylhydrogénosiloxane à 4% de Si-H (ABCR, référence PS 124.5, masse molaire ≈ 2000 g/mol).

Après 48 heures de réaction à 110°C, on récupère 1,1 g (rendement quantitatif) d'une huile visqueuse brune correspondant au polysiloxane A.

Polysiloxane B :

Dans un bicol de 25 mL sous argon, on introduit 0,75 g d'allylpyrène. On porte à 110°C et on injecte 5 μL de catalyseur de Karstedt. Après 15 minutes, on introduit 0,5 g de polydiméthylméthylhydrogénosiloxane à 47% de Si-H (ABCR, référence PS 122.5, masse molaire ≈ 1000 g/mol).

Après 48 heures de réaction à 110°C, on récupère 1,25 g (rendement quantitatif) d'une huile visqueuse brune correspondant au polysiloxane B.

Polysiloxane C :

Dans un bicol de 25 mL sous argon, on introduit 0,5 g d'allylpyrène et 5 mL de toluène. On porte à 110°C et on injecte 5 μL de catalyseur de Karstedt. Après quelques minutes, on introduit 0,22 g de polyméthylhydrogénosiloxane (ABCR, référence PS 118, masse molaire ≈ 400 g/mole).

Après 48 heures de réaction à 110°C, le toluène est évaporé et une purification sur colonne de gel de silice (éluant : pentane/dichlorométhane) est réalisée.

On récupère ainsi 0,35 g (rendement quantitatif) d'une huile visqueuse brune correspondant au polysiloxane C.

**Exemple 2 : Synthèse d'un polysiloxane fluorescent comprenant un motif répétitif de formule générale (I)**

On synthétise un polysiloxane désigné ci-après polysiloxane D et correspondant à un mélange d'oligomères à motif répétitif de formule (III) ci-avant, en soumettant du 1,8-diallylpyrène, préalablement obtenu à partir de 1,8-dibromopyrène, à une hydrosilylation par l'hexaméthyltrisiloxane en présence du catalyseur de Karstedt.
Le protocole opératoire suivi est le suivant.

a) Synthèse du 1,8-dibromopyrène :

Dans un bicol de 250 mL sous argon, on introduit 6,1 g de pyrène (disponible chez SIGMA-ALDRICH sous la référence 13,159-8) et 150 mL de tétrachlorure de carbone. On ajoute une solution de 3,16 mL de brome dans 25 mL de $CCl_4$, puis le mélange est agité à température ambiante pendant 48 heures. Le solide formé est solubilisé à chaud dans le toluène, puis la solution est refroidie et filtrée. On évapore le filtrat et on le recristallise dans un mélange toluène/hexane.
On obtient ainsi 3,15 g de 1,8-dibromopyrène (Rdt : 29%).

b) Synthèse du 1,8-diallylpyrène :

Dans un bicol de 250 mL sous argon, on introduit 24 mL de THF anhydre. La solution est refroidie à 0°C, puis on introduit 6,6 mL d'une solution 2M de BuMgCl dans le THF (SIGMA-ALDRICH, référence 29,100-5).
On ajoute, à 0°C, goutte à goutte 10,56 mL d'une solution 2,5 M de n-butyllithium dans l'hexane (Sigma-Aldrich, référence 23,070-7). Après 30 minutes, 2 g de 1,8-dibromopyrène dans 100 mL de THF anhydre sont alors ajoutés goutte à goutte à 0°C. Le mélange est laissé sous agitation à 0°C pendant 60 minutes. Puis, 4,8 g de bromure d'allyle (SIGMA-ALDRICH, référence A2,958-5) fraîchement distillé sont ajoutés goutte à goutte. Le mélange est laissé sous agitation à 0°C pendant 60 minutes, puis il est ramené et laissé à température ambiante pendant une nuit. Le mélange réactionnel est alors traité par HCl 4N (à 0°C) et extrait avec l'éther diéthylique. La solution extraite est lavée à l'eau, séchée sur $Na_2SO_4$ et concentrée.
On obtient ainsi 1,5 g d'un solide purifié sur colonne de gel de silice (éluant : pentane). Le rendement en produit purifié est de 52%.

c) Hydrosilylation du 1,8-diallylpyrène par l'hexaméthyltrisiloxane :

Dans un bicol de 25 mL sous argon, on introduit 0,5 g de 1,8-diallylpyrène et 5 g de toluène. On porte la solution à 110°C et 5 μL de catalyseur de Karstedt sont alors injectés. Dix minutes plus tard, on introduit goutte à goutte 0,74 g d'hexaméthyltrisiloxane (ABCR). Après 67 heures de réaction et élimination des produits volatils à 110°C sous 0,1 mm Hg, on obtient de manière quantitative un mélange de composés comprenant un, deux ou trois motifs de formule (IV) et terminés par des groupes -Si-H.

**Exemple 3 : Détection d'un composé nitré (DNTFMB) par un capteur à fluorescence comprenant un film mince d'un polysiloxane fluorescent**

Dans cet exemple, on utilise un capteur dont le fonctionnement est basé sur la variation de l'intensité de la fluorescence émise par le matériau sensible que comporte ce capteur en présence d'un composé nitré.
En l'espèce, le matériau sensible est constitué par le polysiloxane C tel que synthétisé dans l'exemple 1, sous la forme d'un film mince qui recouvre l'une des faces d'un substrat en verre de qualité optique (THUET et BIECHELIN).
Ce film mince est obtenu par 3 pulvérisations de 0,1 seconde chacune d'une solution de polysiloxane C dans le chloroforme, de concentration égale à 5 g/L.
Il présente une intensité de fluorescence de $1{,}5.10^7$ coups ($\lambda_{émission}$ : 476 nm ; $\lambda_{excitation}$ : 347 nm).
Le capteur est exposé successivement à :

- de l'air pur pendant 900 secondes,
- du DNTFMB à une concentration de 1 ppm dans de l'air pendant 600 secondes,
- de l'air pur pendant 1900 secondes,
- du DNTFMB à une concentration de 0,1 ppm dans de l'air pendant 600 secondes, et
- de l'air pur pendant 2100 secondes, l'air et le DNTFMB étant à température ambiante (25°C).

La figure 1 illustre l'évolution de l'intensité de la fluorescence émise par le capteur au cours de ces expositions, cette intensité de fluorescence étant mesurée en utilisant les mêmes longueurs d'onde que celles précisées ci-avant.

Sur cette figure, la courbe A représente les valeurs de l'intensité de la fluorescence (I), exprimées en coups (cps), en fonction du temps, exprimé en secondes (s), tandis que la courbe B représente les valeurs de la concentration en DNTFMB (C), exprimées en ppm, également en fonction du temps.

**Exemple 4 : Mise en évidence de la sélectivité vis-à-vis des composés nitrés d'un capteur à fluorescence comprenant un film mince d'un polysiloxane fluorescent**

Dans cet exemple, on expose successivement un capteur identique à celui utilisé dans l'exemple 3 à :

- de l'air pur pendant 900 secondes,
- du DNTFMB à une concentration de 1 ppm dans de l'air pendant 600 secondes,
- de l'air pur pendant 1900 secondes,
- du DNTFMB à une concentration de 0,1 ppm dans de l'air pendant 600 secondes,
- de l'air pur pendant 3000 secondes,
- du dichlorométhane à une concentration de 675 ppm dans de l'air pendant 600 secondes,
- de l'air pur pendant 1500 secondes,
- du cyclohexane à une concentration de 540 ppm dans de l'air pendant 600 secondes,
- de l'air pur pendant 1500 secondes,
- de la méthyléthylcétone à une concentration de 360 ppm dans de l'air pendant 600 secondes,
- de l'air pur pendant 1500 secondes,
- du toluène à une concentration de 180 ppm dans de l'air pendant 600 secondes,
- de l'air pur pendant 1500 secondes,
- de la méthylisobutylcétone à une concentration de 90 ppm dans de l'air pendant 600 secondes,
- de l'air pur pendant 1500 secondes,
- de l'acétate d'éthyle à une concentration de 720 ppm dans de l'air pendant 600 secondes, et
- de l'air pur pendant 2500 secondes, l'air, le DNTFMB et les différents solvants utilisés étant à température ambiante (≈ 25°C).

La figure 2 illustre l'évolution de l'intensité de la fluorescence (I) émise par le capteur au cours de ces expositions, sous la forme d'une courbe représentant les valeurs de l'intensité de la fluorescence (I), exprimées en coups, en fonction du temps, exprimé en secondes (s), les flèches f1 et f2 signalant le début des deux expositions au DNTFMB, la flèche f3 le début de l'exposition au dichlorométhane et la flèche f4 la fin de l'exposition à l'acétate d'éthyle.

**Exemple 5 : Détection d'un composé nitré (DNTFMB) par des capteurs à fluorescence comprenant un film mince de pyrène ou d'un polysiloxane fluorescent**

Dans cet exemple, on compare l'aptitude à détecter des vapeurs de DNTFMB de trois capteurs à fluorescence comprenant le même substrat en verre de qualité optique (THUET et BIECHELIN) mais se différenciant par le film mince qui recouvre ce substrat, le premier de ces capteurs comprenant un film mince de pyrène, le second un film mince de polysiloxane A, et le troisième un film mince de polysiloxane C.

Ces films minces ont été obtenus par pulvérisation de solutions, respectivement de polysiloxane A, de polysiloxane C et de pyrène, à 5 g/l dans le chloroforme, suivie d'une évaporation à température ambiante et sous pression atmosphérique, de façon à conduire aux caractéristiques présentées dans le tableau 1 ci-après.

**TABLEAU 1**

| Matériau sensible | $\lambda_{émission}$ (nm) | $\lambda_{excitation}$ (nm) | Intensité de fluorescence (cps) |
|---|---|---|---|
| Pyrène | 466 | 338 | $4.10^7$ |
| Polysiloxane A | 483 | 343 | $10^7$ |
| Polysiloxane C | 476 | 347 | $1,5.10^7$ |

Les capteurs ont été exposés à du DNTFMB à une concentration de 1 ppm dans de l'air pendant 10 minutes.

On détermine la sensibilité (S) de chacun de ces capteurs par la formule suivante :

$$S = \frac{I_{t10} - I_{t0}}{I_{t0}} \times 100$$

dans laquelle $I_{t0}$ représente l'intensité de la fluorescence émise par un capteur avant exposition au DNTFMB (cette valeur étant indiquée dans le tableau 1), tandis que $I_{t10}$ représente l'intensité de fluorescence émise par ce même capteur au terme des 10 minutes d'exposition au DNTFMB.

La figure 3 représente, sous la forme d'histogrammes, les valeurs de sensibilité ainsi obtenues pour les trois capteurs.

**Exemple 6 : Détection d'un composé nitré (DNTFMB) par un capteur à fluorescence comprenant un film mince d'un polysiloxane fluorescent**

Dans cet exemple, on utilise un capteur à fluorescence comprenant un substrat en verre de qualité optique (THUET et BIECHELIN), recouvert sur l'une de ses faces d'un film mince de polysiloxane D tel que synthétisé dans l'exemple 2.

Ce film mince est obtenu par 2 pulvérisations de 0,2 seconde chacune d'une solution de polysiloxane D dans le chloroforme, de concentration égale à 5 g/L.

Il présente une intensité de fluorescence de $8{,}7.10^6$ coups ($\lambda_{émission}$ : 485 nm ; $\lambda_{excitation}$ : 352 nm).

Le capteur est exposé successivement à :

- de l'air pur pendant 2600 secondes,
- du DNTFMB à une concentration de 1 ppm dans de l'air pendant 600 secondes, et
- de l'air pur pendant 1800 secondes, l'air et le DNTFMB étant à température ambiante (≈ 25°C).

La figure 4 illustre l'évolution de l'intensité de la fluorescence émise par le capteur au cours de ces expositions, cette intensité de fluorescence étant mesurée en utilisant les mêmes longueurs d'onde que celles mentionnées ci-avant.

Sur cette figure, la courbe A représente les valeurs de l'intensité de la fluorescence (I), exprimées en coups (cps), en fonction du temps, exprimé en secondes (s), tandis que la courbe B représente les valeurs de la concentration en DNTFMB (C), exprimées en ppm, également en fonction du temps.

**Exemple 7 : Détection de deux composés nitrés (DNT et DNB) par un capteur à fluorescence comprenant un film mince d'un polysiloxane fluorescent**

Dans cet exemple, on utilise un capteur à fluorescence comprenant un substrat en verre de qualité optique (THUET et BIECHELIN), recouvert sur l'une de ses faces d'un film mince de polysiloxane B.

Ce film mince est obtenu par 5 pulvérisations de 0,5 seconde chacune d'une solution de polysiloxane B dans le chloroforme, de concentration égale à 5 g/L.

Il présente une intensité de fluorescence de $9{,}5.10^6$ coups ($\lambda_{émission}$ : 503 nm ; $\lambda_{excitation}$ : 335 nm).

Le capteur est exposé successivement à :

- de l'air pur pendant 4200 secondes,
- du DNT à une concentration de 286 ppb dans de l'air pendant 600 secondes,
- de l'air pur pendant 7800 secondes,
- du DNB à une concentration de 5 ppm dans de l'air pendant 600 secondes, et
- de l'air pur pendant 1800 secondes, l'air, le DNT et le DNB étant à température ambiante (≈ 25°C).

La figure 5 illustre l'évolution de l'intensité de la fluorescence (I) émise par le capteur au cours de ces expositions, sous la forme d'une courbe représentant les valeurs de l'intensité de la fluorescence (I), exprimées en coups, en fonction du temps, exprimé en secondes (s), la flèche f1 signalant le début de l'exposition au DNT et la flèche f2 signalant le début de l'exposition au DNB.

**Exemple 8 : Détection d'un composé nitré (DNTFMB) par un capteur à microbalance à quartz comprenant un film mince d'un polysiloxane fluorescent**

Dans cet exemple, on utilise un capteur à microbalance à quartz comprenant un quartz de coupe AT, de

fréquence de vibration de 9 MHz, recouvert de deux électrodes de mesure circulaires en or (modèle QA9RA-50, AMETEK PRECISION INSTRUMENTS) et portant sur ses deux faces un film mince de polysiloxane D.

Ces films minces sont obtenus en effectuant sur chaque face du quartz 10 pulvérisations de 0,3 seconde chacune d'une solution de polysiloxane D dans du chloroforme, de concentration égale à 10 g/L.

Leur formation sur le quartz se traduit par une variation de fréquence de ce quartz de 2 kHz.

Le capteur est exposé successivement à :

- l'air ambiant pendant 2300 secondes,
- du DNTFMB à une concentration de 3 ppm pendant 600 secondes, et
- l'air ambiant pendant 800 secondes, l'air et le DNTFMB étant à température ambiante (≈ 25°C).

La figure 6 montre l'évolution de la fréquence de vibration du quartz au cours de ces expositions, la courbe A représentant les valeurs de la fréquence (F) de vibration du quartz, exprimées en hertz (Hz), en fonction du temps, exprimé en secondes (s), tandis que la courbe B représente les valeurs de la concentration en DNTFMB (C), exprimées en ppm, également en fonction du temps.

Les exemples 3 à 8 ci-avant montrent que des polysiloxanes fluorescents comprenant un motif répétitif répondant à l'une des formules (I) et (II) telles que précédemment définies permettent, lorsqu'ils sont utilisés comme matériaux sensibles dans des capteurs chimiques, de détecter avec une très grande sensibilité des composés nitrés comme le DNTFMB, le DNT ou le DNB et ce, que ces capteurs soient à fluorescence ou à microbalance à quartz.

L'exemple 6 montre, de plus, que la sensibilité d'un capteur chimique ayant pour matériau sensible, un polysiloxane fluorescent comprenant un motif de formule générale (I) ou (II) est nettement plus élevée que celle d'un capteur chimique de structure identique mais ne comprenant que le fluorophore présent dans ce polysiloxane en tant que matériau sensible, tandis que l'exemple 4 montre que cette sensibilité s'accompagne d'une spécificité vis-à-vis des composés nitrés.

Enfin, ces exemples montrent que la réponse des capteurs est à la fois réversible et reproductible.

**REFERENCES CITEES**

**[1]** McGill et al., Sensors and Actuators B65, 5-9, 2000

**[2]** WO-A-98/22795

**[3]** B. Valeur, Molecular Fluorescence : Principles and Applications, 2002, Ed. WILEY VCH, New York

**[4]** J.A.O. Sanchez-Pedrono et al., Anal. Chem. Acta, 182, 1986, 285


**Revendications**

1. Utilisation d'au moins un polysiloxane fluorescent comprenant un motif répétitif siloxane répondant à l'une des formules (I) et (II) ci-après :

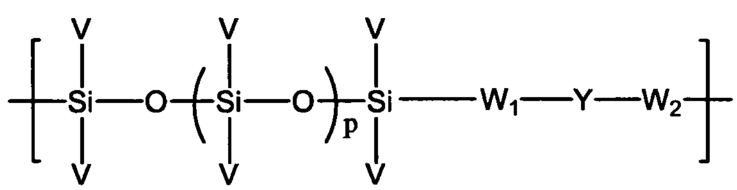

(I)

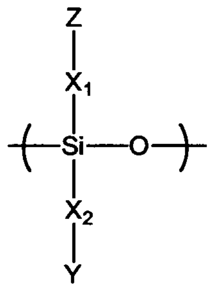

(II)

dans lesquelles :

$W_1$ et $W_2$, qui peuvent être identiques ou différents, représentent un groupe hydrocarboné comprenant de 2 à 50 atomes de carbone et comportant éventuellement un ou plusieurs hétéroatomes et/ou une ou plusieurs fonctions chimiques comprenant au moins un hétéroatome ;
V représente un atome d'hydrogène ou un groupe hydrocarboné comprenant de 1 à 50 atomes de carbone et comportant éventuellement un ou plusieurs hétéroatomes et/ou une ou plusieurs fonctions chimiques comprenant au moins un hétéroatome ;
p est un nombre entier allant de 0 à 10 000 ;
Y représente un groupe fluorophore ;
$X_1$ et $X_2$, qui peuvent être identiques ou différents, représentent une liaison simple ou un groupe hydrocarboné, comprenant de 1 à 50 atomes de carbone et comportant éventuellement un ou plusieurs hétéroatomes et/ou une ou plusieurs fonctions chimiques comprenant au moins un hétéroatome ; et
Z représente un atome d'hydrogène ou a la même signification que Y ;

en tant que matériau sensible dans un capteur chimique pour la détection ou le dosage d'au moins un composé nitré.

**2.** Utilisation selon la revendication 1, dans laquelle le motif répétitif siloxane répond à l'une des formules générales (I) et (II) dans lesquelles Y représente un groupe fluorophore choisi parmi les groupes naphtacényle, naphtalényle, acénaphtyle, dansyle, xanthanyle, thioxanthanyle, anthraquinyle, acridinyle, rhodaminyle, fluorényle, fluoranthrényle, pentacényle, tétrahydrochrysényle, carbazolyle, pyridyle, imidazolyle, phénothiazolyle, toluidinyle, 1H-benzimidazo-[2,1-a]benz[de]isoquinoléinyle, 4-(2'-phényl)vinylphényle, anthracényle, pyrényle, pérylényle, benzopérylényle, benzo[k]-fluoranthényle, benzothiazolyle, benzimidazolyle, oligo(phénylène-vinylidényle), oligo-(phénylèneéthynylényle), fluorescéinyle, coumarinyle, pyridyloxazolyle, benzoxazolyle, benzoxadiazolyle, 5,5'-bis(4-amino-phényl)-2,2'-bifuryle et les dérivés fonctionnels de ceux-ci.

**3.** Utilisation selon la revendication 3, dans laquelle le motif répétitif siloxane répond à l'une des formules générales (I) et (II) dans laquelle Y est un groupe pyrényle.

**4.** Utilisation selon l'une quelconque des revendications précédentes, dans laquelle le motif répétitif siloxane répond à la formule générale (I) dans laquelle $W_1$ et $W_2$ représentent, indépendamment l'un de l'autre, un groupe alkylène comprenant de 2 à 10 atomes de carbone, V représente un groupe alkyle comportant de 1 à 10 atomes de carbone, tandis que p et Y sont tels que précédemment définis.

**5.** Utilisation selon la revendication 4, dans laquelle le motif répétitif siloxane répond à la formule générale (I) dans laquelle $W_1$ et $W_2$ sont tous les deux un groupe propylène, V est un groupe méthyle, p vaut 1 et Y est tel que précédemment défini.

**6.** Utilisation selon la revendication 5, dans laquelle le motif répétitif siloxane répond à la formule (III) ci-après :

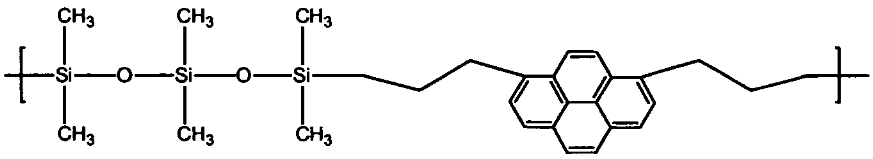

(III)

7. Utilisation selon l'une quelconque des revendications 1 à 3, dans laquelle le motif répétitif siloxane répond à la formule générale (II) dans laquelle $X_1$ représente un groupe alkylène comprenant de 1 à 10 atomes de carbone, $X_2$ représente un groupe alkylène comprenant de 2 à 10 atomes de carbone, Z est un atome d'hydrogène, tandis que Y est tel que précédemment défini.

8. Utilisation selon la revendication 7, dans laquelle le motif répétitif siloxane répond à la formule générale (II) dans laquelle $X_1$ est un groupe méthylène, $X_2$ est un groupe propylène, Z est un atome d'hydrogène, tandis que Y est tel que précédemment défini.

9. Utilisation selon la revendication 8, dans laquelle le motif répétitif siloxane répond à la formule (IV) ci-après :

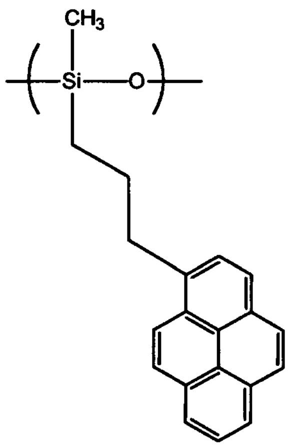

(IV)

10. Utilisation selon l'une quelconque des revendications précédentes, dans laquelle le polysiloxane fluorescent est un homopolymère.

11. Utilisation selon les revendications 6 et 10, dans laquelle le polysiloxane fluorescent est un homopolymère à motif répétitif siloxane de formule (III) .

12. Utilisation selon les revendications 9 et 10, dans laquelle le polysiloxane fluorescent est un homopolymère à motif répétitif siloxane de formule (IV).

13. Utilisation selon l'une quelconque des revendications 1 à 9, dans laquelle le polysiloxane fluorescent est un copolymère constitué d'un premier motif répétitif siloxane répondant à l'une des formules générales (I) et (II) dans lesquelles $W_1$, $W_2$, V, p, Y, $X_1$, $X_2$ et Z sont tels que précédemment définis, et d'un deuxième motif répétitif siloxane répondant à la formule (V) ci-après :

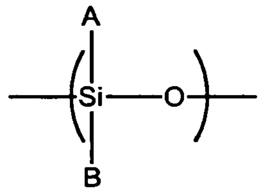

(V)

dans laquelle A et B, qui peuvent être identiques ou différents, représentent un atome d'hydrogène ou un groupe hydrocarboné, saturé ou insaturé, comprenant de 1 à 50 atomes de carbone et comportant éventuellement un ou plusieurs hétéroatomes et/ou une ou plusieurs fonctions chimiques comprenant au moins un hétéroatome.

**14.** Utilisation selon la revendication 13, dans laquelle le deuxième motif répétitif siloxane est un motif diméthylsiloxane.

**15.** Utilisation selon les revendications 6 et 14, dans laquelle le polysiloxane fluorescent est un copolymère constitué d'un premier motif répétitif siloxane répondant à la formule (IV) et d'un deuxième motif répétitif diméthylsiloxane.

**16.** Utilisation selon l'une quelconque des revendications 13 à 15, dans laquelle le copolymère est aléatoire, alterné ou séquencé.

**17.** Utilisation selon l'une quelconque des revendications précédentes, dans laquelle le polysiloxane fluorescent est présent sous la forme d'un film mince recouvrant l'une ou les deux faces d'un substrat.

**18.** Utilisation selon la revendication 17, dans laquelle le film mince mesure de 10 angströms à 100 microns d'épaisseur.

**19.** Utilisation selon l'une quelconque des revendications précédentes, dans laquelle le capteur est un capteur optique à fluorescence.

**20.** Utilisation selon l'une quelconque des revendications 1 à 19, dans laquelle le capteur est un capteur gravimétrique, de préférence à microbalance à quartz.

**21.** Utilisation selon l'une quelconque des revendications 1 à 18, dans laquelle le capteur est un multicapteur qui comprend un ou plusieurs capteurs optiques à fluorescence et/ou un ou plusieurs capteurs gravimétriques, l'un au moins de ces capteurs comprenant au moins un polysiloxane fluorescent tel que précédemment défini, en tant que matériau sensible.

**22.** Utilisation selon l'une quelconque des revendications précédentes, dans laquelle le composé nitré est choisi parmi les composés nitroaromatiques, les nitramines, les nitrosamines et les esters nitriques.

**23.** Utilisation selon la revendication 22, dans laquelle le composé nitré est choisi parmi le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'aminodinitrotoluène, le dinitrotrifluorométhylbenzène, le chlorodinitrotrifluorométhylbenzène, l'hexanitrostilbène, le trinitrophénol, la cyclotétraméthylènetétranitramine, la cyclotriméthylènetrinitramine, la trinitrophénylméthylnitramine, la nitrosodiméthylamine, le pentrite, le dinitrate d'éthylène glycol, le dinitrate de diéthylène glycol, la nitroglycérine ou la nitroguanidine.

**24.** Utilisation selon l'une quelconque des revendications précédentes pour la détection d'explosifs.

**25.** Polysiloxane fluorescent qui comprend un motif répétitif répondant à la formule générale (I) ci-après :

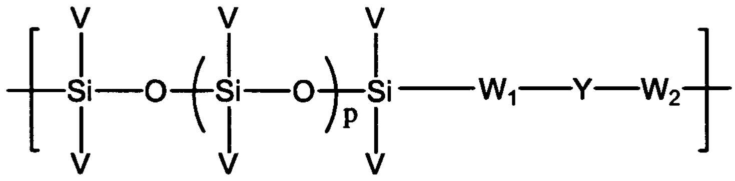

(I)

dans laquelle :

$W_1$ et $W_2$, qui peuvent être identiques ou différents, représentent un groupe hydrocarboné comprenant de 2 à 50 atomes de carbone et comportant éventuellement un ou plusieurs hétéroatomes et/ou une ou plusieurs fonctions chimiques comprenant au moins un hétéroatome ;
V représente un atome d'hydrogène ou un groupe hydrocarboné comprenant de 1 à 50 atomes de carbone et comportant éventuellement un ou plusieurs hétéroatomes et/ou une ou plusieurs fonctions chimiques comprenant au moins un hétéroatome ;
p est un nombre entier allant de 0 à 10 000 ; et
Y représente un groupe fluorophore.

**26.** Polysiloxane fluorescent selon la revendication 25, qui comprend un motif répétitif siloxane répondant à la formule générale (II) dans laquelle Y représente un groupe fluorophore choisi parmi les groupes naphtacényle, naphtalényle, acénaphtyle, dansyle, xanthanyle, thioxanthanyle, anthraquinyle, acridinyle, rhodaminyle, fluorényle, fluoranthrényle, pentacényle, tétrahydrochrysényle, carbazolyle, pyridyle, imidazolyle, phénothiazolyle, toluidinyle, 1H-benzimidazo-[2,1-a]benz[de]isoquinoléinyle, 4-(2'-phényl)vinylphényle, anthracényle, pyrényle, pérylényle, benzopérylényle, benzo[k]-fluoranthényle, benzothiazolyle, benzimidazolyle, oligo(phénylène-vinylidényle), oligo-(phénylèneéthynylényle), fluorescéinyle, coumarinyle, pyridyloxazolyle, benzoxazolyle, benzoxadiazolyle, 5,5'-bis(4-aminophényl)-2,2'-bifuryle et les dérivés fonctionnels de ceux-ci.

**27.** Polysiloxane fluorescent selon la revendication 25 ou la revendication 26, qui comprend un motif répétitif siloxane répondant à la formule générale (I) dans laquelle Y est un groupe pyrényle.

**28.** Polysiloxane fluorescent selon l'une quelconque des revendications 25 à 27, qui comprend un motif répétitif répondant à la formule générale (I) dans laquelle $W_1$ et $W_2$ représentent, indépendamment l'un de l'autre, un groupe alkylène comportant de 2 à 10 atomes de carbone, V représente un groupe alkyle comportant de 1 à 10 atomes de carbone, tandis que p et Y sont tels que précédemment définis.

**29.** Polysiloxane fluorescent selon la revendication 28, qui comprend un motif répétitif siloxane répondant à la formule générale (I) dans laquelle $W_1$ et $W_2$ sont tous les deux un groupe propylène, V est un groupe méthyle, p vaut 1 et Y est tel que précédemment défini.

**30.** Polysiloxane fluorescent selon la revendication 29, qui comprend un motif répétitif siloxane répondant à la formule (III) ci-après :

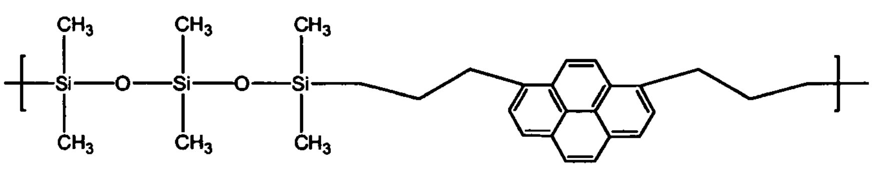

(III)

**31.** Polysiloxane fluorescent selon l'une quelconque des revendications 25 à 30, qui est un homopolymère.

**32.** Polysiloxane fluorescent selon la revendication 30 ou la revendication 31, qui est un homopolymère à motif répétitif siloxane de formule (III) .

**33.** Capteur chimique qui comprend au moins un polysiloxane fluorescent tel que défini dans l'une quelconque des revendications 25 à 32, comme matériau sensible.

**34.** Capteur chimique selon la revendication 33, dans lequel le polysiloxane fluorescent est présent sous la forme d'un film mince recouvrant l'une ou les deux faces d'un substrat.

**35.** Capteur selon la revendication 34, dans lequel le film mince mesure de 10 angströms à 100 microns d'épaisseur.

**36.** Capteur selon l'une quelconque des revendications 33 à 35, dans lequel le capteur est un capteur optique à fluorescence.

**37.** Capteur selon l'une quelconque des revendications 33 à 35, dans lequel le capteur est un capteur gravimétrique, de préférence à microbalance à quartz.

**38.** Capteur selon l'une quelconque des revendications 33 à 35, dans lequel le capteur est un multicapteur qui comprend un ou plusieurs capteurs optiques à fluorescence et/ou un ou plusieurs capteurs gravimétriques, l'un au moins de ces capteurs comprenant au moins un polysiloxane fluorescent tel que précédemment défini.

**39.** Procédé de préparation d'un polysiloxane fluorescent constitué d'un motif répétitif répondant à la formule générale (I) ci-après :

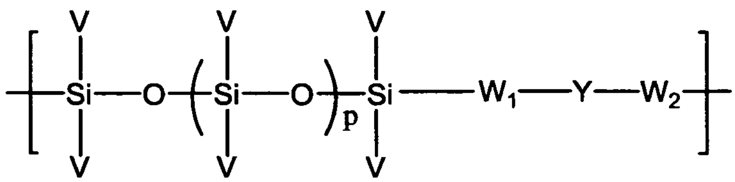

(I)

dans laquelle :

$W_1$ et $W_2$, qui peuvent être identiques ou différents, représentent un groupe hydrocarboné comprenant de 2 à 50 atomes de carbone et comportant éventuellement un ou plusieurs hétéroatomes et/ou une ou plusieurs fonctions chimiques comprenant au moins un hétéroatome ;
V représente un atome d'hydrogène ou un groupe hydrocarboné comprenant de 1 à 50 atomes de carbone et comportant éventuellement un ou plusieurs hétéroatomes et/ou une ou plusieurs fonctions chimiques comprenant au moins un hétéroatome ;
p est un nombre entier allant de 0 à 10 000 ; et
Y représente un groupe fluorophore ;

lequel procédé comprend la polycondensation d'un composé de formule (VI) ci-après :

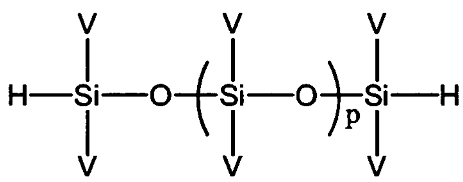

(VI)

dans laquelle V et p ont les mêmes significations que dans la formule générale (I), avec un composé de formule (VII) ci-après :

$$\mathbf{P_1—Y—P_2} \qquad \text{(VII)}$$

dans laquelle :

Y a la même signification que dans la formule générale (I) ; et

$P_1$ et $P_2$, qui peuvent être identiques ou différents, représentent un groupe hydrocarboné qui comprend de 2 à 50 atomes de carbone et qui est terminé par un groupe $-CH=CH_2$.

**40.** Procédé de préparation d'un polysiloxane fluorescent constitué de plusieurs motifs répétitifs siloxane différents, dont l'un au moins répond à la formule générale (I) ci-après :

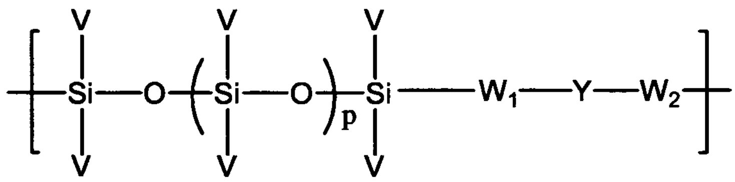

(I)

dans laquelle :

$W_1$ et $W_2$, qui peuvent être identiques ou différents, représentent un groupe hydrocarboné comprenant de 2 à 50 atomes de carbone et comportant éventuellement un ou plusieurs hétéroatomes et/ou une ou plusieurs fonctions chimiques comprenant au moins un hétéroatome ;

V représente un atome d'hydrogène ou un groupe hydrocarboné comprenant de 1 à 50 atomes de carbone et comportant éventuellement un ou plusieurs hétéroatomes et/ou une ou plusieurs fonctions chimiques comprenant au moins un hétéroatome ;

p est un nombre entier allant de 0 à 10 000 ; et

Y représente un groupe fluorophore ;

lequel procédé comprend la polycondensation d'un monomère de formule (VIII) ci-après:

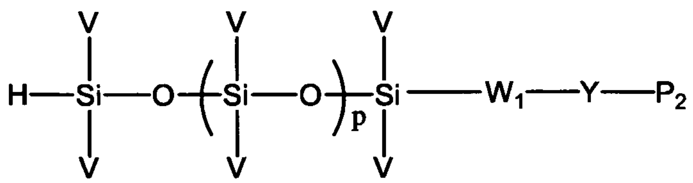

(VIII)

dans laquelle :

$W_1$, V, Y et p ont les mêmes significations que dans la formule générale (I) ; et

$P_2$ représente un groupe hydrocarboné qui comprend de 2 à 50 atomes de carbone et qui est terminé par un groupe $-CH=CH_2$ ;

ou d'un oligomère résultant de la condensation de plusieurs monomères de formule (VIII), avec un autre monomère siloxane ou un autre oligomère formé à partir de ce dernier monomère.

**Claims**

**1.** Use of at least one fluorescent polysiloxane comprising a siloxane repeat unit corresponding to either of the formulae

(I) and (II) below:

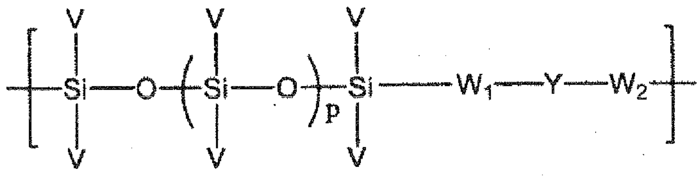

(I)

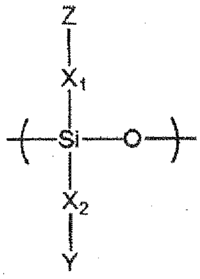

(II)

in which:

$W_1$ and $W_2$, which can be identical or different, represent a hydrocarbon group comprising from 2 to 50 carbon atoms and optionally comprising one or more heteroatoms and/or one or more chemical functional groups comprising at least one heteroatom;
V represents a hydrogen atom or a hydrocarbon group comprising from 1 to 50 carbon atoms and optionally comprising one or more heteroatoms and/or one or more chemical functional groups comprising at least one heteroatom;
is an integer ranging from 0 to 10 000;
Y represents a fluorophoric group;
$X_1$ and $X_2$, which can be identical or different, represent a single bond or a hydrocarbon group comprising from 1 to 50 carbon atoms and optionally comprising one or more heteroatoms and/or one or more chemical functional groups comprising at least one heteroatom; and
Z represents a hydrogen atom or has the same meaning as Y;
as sensitive material in a chemical sensor for the detection or the assaying of at least one nitrogenous compound.

**2.** Use according to Claim 1, in which the siloxane repeat unit corresponds to either of the general formulae (I) and (II) in which Y represents a fluorophoric group chosen from naphthacenyl, naphthalenyl, acenaphthyl, dansyl, xanthanyl, thioxanthanyl, anthraquinyl, acridinyl, rhodaminyl, fluorenyl, fluoranthenyl, pentacenyl, tetrahydrochrysenyl, carbazolyl, pyridyl, imidazolyl, phenothiazolyl, toluidinyl, 1H-benzimidazo [2,1-a]benz[de]isoquinolinyl, 4-(2'-phenyl)vinylphenyl, anthracenyl, pyrenyl, perylenyl, benzoperylenyl, benzo[k]fluoranthenyl, benzothiazolyl, benzimidazolyl, oligo (phenylene-vinylidenyl), oligo(phenylene-ethynylenyl), fluorescenyl, coumarinyl, pyridyloxazolyl, benzoxazolyl, benzoxadiazolyl or 5,5'-bis(4-aminophenyl)-2,2'-bifuryl groups and the functional derivatives of these.

**3.** Use according to Claim 3, in which the siloxane repeat unit corresponds to either of the general formulae (I) and (II) in which Y is a pyrenyl group.

**4.** Use according to any one of the preceding claims, in which the siloxane repeat unit corresponds to the general formula (I) in which $W_1$ and $W_2$ represent, independently of one another, an alkylene group comprising from 2 to 10 carbon atoms, V represents an alkyl group comprising from 1 to 10 carbon atoms, while p and Y are as defined above.

**5.** Use according to Claim 4, in which the siloxane repeat unit corresponds to the general formula (I) in which $W_1$ and

$W_2$ are both a propylene group, V is a methyl group, p has the value 1 and Y is s as defined above.

6. Use according to Claim 5, in which the siloxane repeat unit corresponds to the formula (III) below:

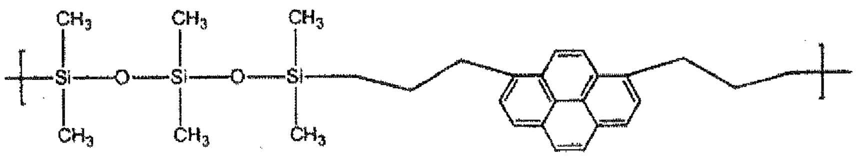

(III)

7. Use according to any one of Claims 1 to 3, in which the siloxane repeat unit corresponds to the general formula (II) in which $X_1$ represents an alkylene group comprising from 1 to 10 carbon atoms, $X_2$ represents an alkylene group comprising from 2 to 10 carbon atoms, Z is a hydrogen atom, while Y is as defined above.

8. Use according to Claim 7, in which the siloxane repeat unit corresponds to the general formula (II) in which $X_1$ is a methylene group, $X_2$ is a propylene group, Z is a hydrogen atom, while Y is as defined above.

9. Use according to Claim 8, in which the siloxane repeat unit corresponds to the formula (IV) below:

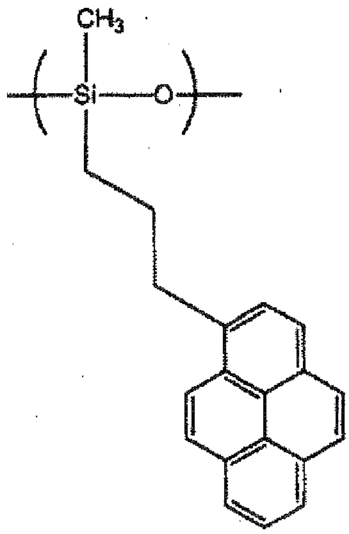

(IV)

10. Use according to any one of the preceding claims, in which the fluorescent polysiloxane is a homopolymer.

11. Use according to Claims 6 and 10, in which the fluorescent polysiloxane is a homopolymer comprising a siloxane repeat unit of formula (III).

12. Use according to Claims 9 and 10, in which the fluorescent polysiloxane is a homopolymer comprising a siloxane repeat unit of formula (IV).

13. Use according to any one of Claims 1 to 9, in which the fluorescent polysiloxane is a copolymer composed of a first siloxane repeat unit corresponding to one of the general formulae (I) and (II) in which $W_1$, $W_2$, V, p, Y, $X_1$, $X_2$ and Z are as defined above and of a second siloxane repeat unit corresponding to the formula (V) below:

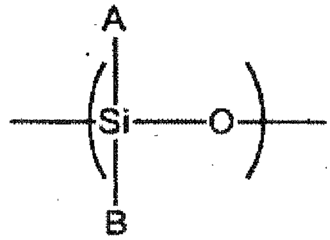

(V)

in which A and B, which can be identical or different, represent a hydrogen atom or a saturated or unsaturated hydrocarbon group comprising from 1 to 50 carbon atoms and optionally comprising one or more heteroatoms and/or one or more chemical functional groups comprising at least one heteroatom.

**14.** Use according to Claim 13, in which the second siloxane repeat unit is a dimethylsiloxane unit.

**15.** Use according to Claims 6 and 14, in which the fluorescent polysiloxane is a copolymer composed of a first siloxane repeat unit corresponding to the formula (IV) and of a second dimethylsiloxane repeat unit.

**16.** Use according to any one of Claims 13 to 15, in which the copolymer is random, alternating or block.

**17.** Use according to any one of the preceding claims, in which the fluorescent polysiloxane is present in the form of a thin film covering one or both faces of a substrate.

**18.** Use according to Claim 17, in which the thin film measures from 10 angstroms to 100 microns in thickness.

**19.** Use according to any one of the preceding claims, in which the sensor is a fluorescence optical sensor.

**20.** Use according to any one of Claims 1 to 19, in which the sensor is a gravimetric sensor, preferably a quartz microbalance sensor.

**21.** Use according to any one of Claims 1 to 18, in which the sensor is a multisensor which comprises one or more fluorescence optical sensors and/or one or more gravimetric sensors, at least one of these sensors comprising at least one fluorescent polysiloxane as defined above as sensitive material.

**22.** Use according to any one of the preceding claims, in which the nitrogenous compound is chosen from nitroaromatic compounds, nitramines, nitrosamines and nitric esters.

**23.** Use according to Claim 22, in which the nitrogenous compound is chosen from nitrobenzene, dinitrobenzene, trinitrobenzene, nitrotoluene, dinitrotoluene, trinitrotoluene, dinitrofluorobenzene, dinitrotrifluoromethoxybenzene, aminodinitrotoluene, dinitrotrifluoromethylbenzene, chlorodinitrotrifluoromethylbenzene, hexanitrostilbene, trinitrophenol, cyclotetramethylenetetranitramine, cyclotrimethylenetrinitramine, trinitrophenylmethylnitramine, nitrosodimethylamine, pentrite, ethylene glycol dinitrate, diethylene glycol dinitrate, nitroglycerine or nitroguanidine.

**24.** Use according to any one of the preceding claims in the detection of explosives.

**25.** Fluorescent polysiloxane which comprises a repeat unit corresponding to the general formula (I) below:

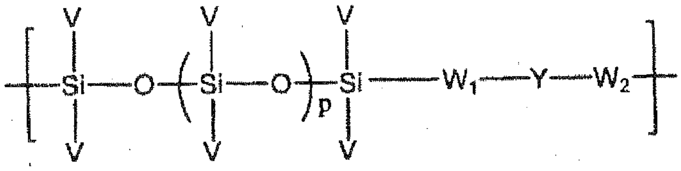

(I)

in which:

$W_1$ and $W_2$, which can be identical or different, represent a hydrocarbon group comprising from 2 to 50 carbon atoms and optionally comprising one or more heteroatoms and/or one or more chemical functional groups comprising at least one Heteroatom;
V represents a hydrogen atom or a hydrocarbon group comprising from 1 to 50 carbon atoms and optionally comprising one or more heteroatoms and/or one or more chemical functional groups comprising at least one heteroatom;
p is an integer ranging from 0 to 10 000; and
Y represents a fluorophoric group.

**26.** Fluorescent polysiloxane according to Claim 25, which comprises a siloxane repeat unit corresponding to the general formula (11) in which Y represents a fluorophoric group chosen from naphthacenyl, hale, acenaphthyl, dansyl, xanthanyl, thioxanthanyl, anthraquinyl, acridinyl, rhodaminyl, fluorenyl, fluoranthenyl, pentacenyl, tetrahydrochrysenyl, carbazolyl, pyridyl, imidazolyl, phenothiazolyl, toluidinyl, 1H-benzimidazo [2,1-a]benz[de]isoquinolinyl, 4-(2'-phenyl)vinylphenyl, anthracenyl, pyrenyl, perylenyl, benzoperylenyl, benzo[k]fluoranthenyl, benzothiazolyl, benzimidazolyl, oligo(phenylene-vinylidenyl), oligo(phenyleneethynylenyl), fluorescenyl, coumarinyl, pyridyloxazolyl, benzoxazolyl, benzoxadiazolyl or 5, 5' -bis ; 4-aminopnenyl)-2,2'-bifuryl groups and the functional derivatives of these.

**27.** Fluorescent polysiloxane according to Claim 25 or Claim 26, which comprises a siloxane repeat unit corresponding to the general formula (I) in which Y is a pyrenyl group.

**28.** Fluorescent polysiloxane according to any one of Claims 25 to 27, which comprises a repeat unit corresponding to the general formula (I) in which $W_1$ and $W_2$ represent, independently of one another, an alkylene group comprising from 2 to 10 carbon atoms, V represents an alkyl group comprising from 1 to 10 carbon atoms, while p and Y are as defined above.

**29.** Fluorescent polysiloxane according to Claim 28, which comprises a siloxane repeat unit corresponding to the general formula (I) in which $W_1$ and $W_2$ are both a propylene group, V is a methyl group, p has the value 1 and Y is as defined above.

**30.** Fluorescent Polysiloxane according to Claim 29, which comprises a siloxane repeat unit corresponding to the formula (III) below :

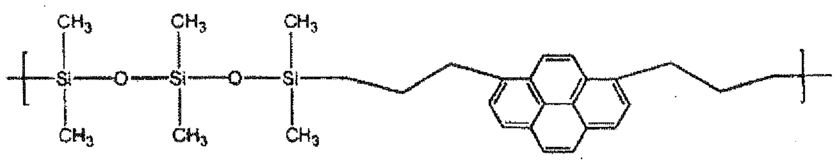

(III)

**31.** Fluorescent polysiloxane according to any one of Claims 25 to 30, which is a homopolymer.

**32.** Fluorescent polysiloxane according to Claim 30 or Claim 31, which is a homopolymer comprising a siloxane repeat unit of formula (III).

**33.** Chemical sensor, which comprises at least one fluorescent polysiloxane as defined in any one of Claims 25 to 32 as sensitive material.

**34.** Chemical sensor according to Claim 33, in which the fluorescent polysiloxane is present in the form of a thin film covering one or both faces of a substrate.

**35.** Sensor according to Claim 34, in which the thin film measures from 10 angstroms to 100 microns in thickness.

**36.** Sensor according to any one of Claims 33 to 35, in which the sensor is a fluorescence optical sensor.

**37.** Sensor according to any one of Claims 33 to 35, in which the sensor is a gravimetric sensor, preferably a quartz microbalance sensor.

**38.** Sensor according to any one of Claims 33 to 35, in which the sensor is a multisensor which comprises one or more fluorescence optical sensors and/or one or more gravimetric sensors, at least one of these sensors comprising at least one fluorescent Polysiloxane as defined above.

**39.** Process for the preparation of a fluorescent polysiloxane composed of a repeat unit corresponding to the general formula (I) below:

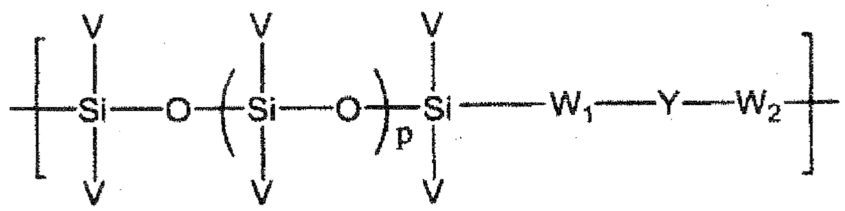

(I)

in which:

$W_1$ and $W_2$, which can be identical or different, represent a hydrocarbon group comprising from 2 to 50 carbon atoms and optionally comprising one or more heteroatoms and/or one or more chemical functional groups comprising at least one heteroatom;
V represents a hydrogen atom or a hydrocarbon group comprising from 1 to 50 carbon atoms and optionally comprising one or more heteroaroms and/or one or more chemical functional groups comprising at least one Heteroatom;
p is an integer ranging from 0 to 10 000; and
Y represents a fluorophoric group;
which process comprises the polycondensation of a compound of formula (VI) below:

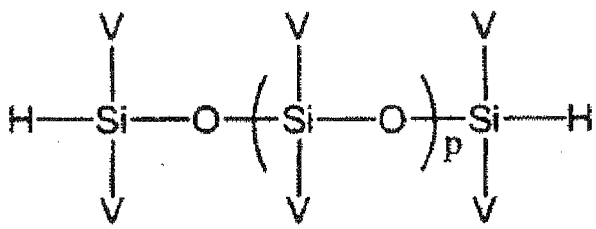

(VI)

in which V and p have the same meanings as in the general formula (I), with a compound of formula (VII) below:

**$P_1$—Y—$P_2$** (VII)

in which:

Y has the same meaning as in the general formula (I); and
$P_1$ and $P_2$, which can be identical or different, represent a hydrocarbon group which comprises from 2 to 50 carbon atoms and which is terminated by a $-CH=CH_2$ group.

**40.** Process for the preparation of a fluorescent polysiloxane composed of several different siloxane repeat units, at least one of which corresponds to the general formula (I) below:

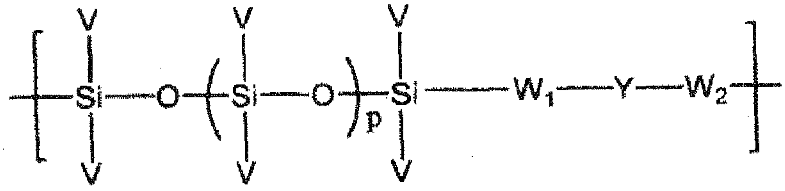

(I)

in which:

$W_1$ and $W_2$, which can be identical or different, represent a hydrocarbon group comprising from 2 to 50 carbon atoms and optionally comprising one or more heteroatoms and/or one or more chemical functional groups comprising at least one heteroatom;
V represents a hydrogen atom or a hydrocarbon group comprising from 1 to 50 carbon atoms and optionally comprising one or more heteroatoms and/or one or more chemical functional groups comprising at least one heteroatom;
p is an integer ranging from 0 to 10 000; and
Y represents a fluorophoric group;

which process comprises the polycondensation of a monomer of formula (VIII) below:

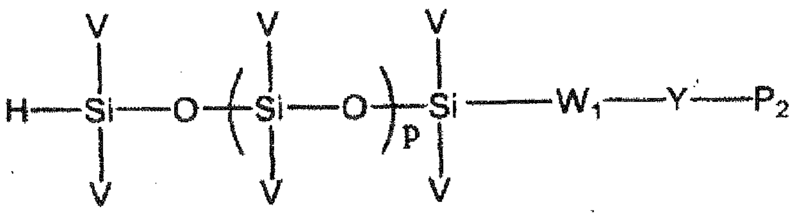

(VIII)

in which:

$W_1$, V, Y and p have the same meanings as in the general formula (I); and
$P_2$ represents a hydrocarbon group which comprises from 2 to 50 carbon atoms and which is terminated by a $-CH=CH_2$ group;
or of an oligomer resulting from the condensation of several monomers of formula (VIII), with another siloxane monomer or another oligomer formed from the latter monomer.

**Patentansprüche**

**1.** Verwendung wenigstens eines fluoreszierenden Polysiloxan, das eine sich wiederholende, einer der nachstehenden Formeln (I) und (II) entsprechende Siloxanstruktureinheit umfasst:

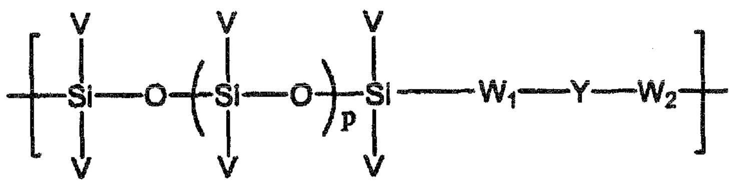

(I)

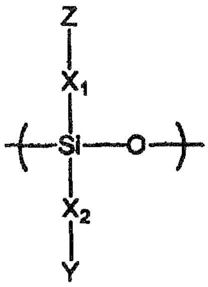

(II)

worin:

$W_1$ und $W_2$, die gleich oder verschieden sein können, eine Kohlenwasserstoffgruppe darstellen, die 2 bis 50 Kohlenstoffatome umfasst und gegebenenfalls ein oder mehrere Heteroatome und/oder eine oder mehrere chemische Funktionen aufweist, die wenigstens ein Heteroatom umfassen;
V ein Wasserstoffatom oder eine Kohlenwasserstoffgruppe darstellt, die 1 bis 50 Kohlenstoffatome umfasst und gegebenenfalls ein oder mehrere Heteroatome und/oder eine oder mehrere chemische Funktionen aufweist, die wenigstens ein Heteroatom umfassen; p eine ganze Zahl von 0 bis 10000 ist;
Y eine fluorophore Gruppe darstellt;
$X_1$ und $X_2$, die gleich oder verschieden sein können, eine Einfachbindung oder eine Kohlenwasserstoffgruppe darstellen, die 1 bis 50 Kohlenstoffatome umfasst und gegebenenfalls ein oder mehrere Heteroatome und/oder eine oder mehrere chemische Funktionen aufweist, die wenigstens ein Heteroatom umfassen; und
Z ein Wasserstoffatom darstellt oder dieselbe Bedeutung wie Y aufweist,
als empfindliches Material in einem chemischen Sensor zum Nachweis oder der Bestimmung wenigstens einer Nitroverbindung.

**2.** Verwendung gemäß Anspruch 1, wobei die sich wiederholende Siloxanstruktureinheit einer der allgemeinen Formeln (I) und (II) entspricht, worin Y eine fluorophore Gruppe darstellt, die aus einer Naphthacenyl-, Naphthalinyl-, Acenaphthyl-, Dansyl-, Xanthanyl-, Thioxanthanyl-, Anthrachinyl-, Acridinyl-, Rhodaminyl-, Fluorenyl-, Fluoranthrenyl-, Pentacenyl-, Tetrahydrochrysenyl-, Carbazolyl-, Pyridyl-, Imidazolyl-, Phenothiazolyl-, Toluidinyl-, 1H-Benzimidazo [2,1-a]benz[de]isochinolinyl-, 4-(2'-Phenyl)vinylphenyl-, Anthracenyl-, Pyrenyl-, Perylenyl-, Benzoperylenyl-, Benzo [k]fluoranthenyl, Benzothiazolyl, Benzimidazolyl-, Oligo(phenylenvinylidenyl-), Oligo(phenylenethinylenyl-), Fluoresceinyl-, Cumarinyl-, Pyridyloxazolyl-, Benzoxazolyl-, Benzoxadiazolyl- und 5,5'-Bis(4-aminophenyl)-2,2'-bifurylgruppe und deren funktionellen Derivaten ausgewählt ist.

**3.** Verwendung gemäß Anspruch 3, wobei die sich wiederholende Siloxanstruktureinheit einer der allgemeinen Formeln (I) und (II) entspricht, worin Y eine Phenylgruppe darstellt.

**4.** Verwendung gemäß einem der vorangehenden Ansprüche, wobei die sich wiederholende Siloxanstruktureinheit der allgemeinen Formeln (I) entspricht, worin $W_1$ und $W_2$ unabhängig von einander eine 2 bis 10 Kohlenstoffatome umfassende Alkylgruppe darstellen, V eine 1 bis 10 Kohlenstoffatome umfassende Alkylgruppe darstellt, während p und Y wie voranstehend definiert sind.

**5.** Verwendung gemäß Anspruch 4, wobei die sich wiederholende Siloxanstruktureinheit der allgemeinen Formel (I) entspricht, worin $W_1$ und $W_2$ alle beide eine Propylengruppe sind, V eine Methylgruppe ist, p 1 ist und Y wie voranstehend definiert ist.

**6.** Verwendung gemäß Anspruch 5, wobei die sich wiederholende Siloxanstruktureinheit der nachstehenden Formel (III) entspricht:

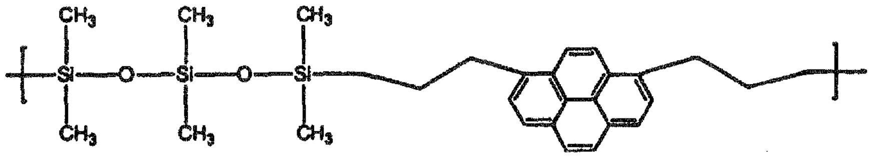

(III)

**7.** Verwendung gemäß einem der Ansprüche 1 bis 3, wobei die sich wiederholende Siloxanstruktureinheit der allgemeinen Formel (II) entspricht, worin $X_1$ eine 1 bis 10 Kohlenstoffatome umfassende Alkylengruppe darstellt, $X_2$ eine 2 bis 10 Kohlenstoffatome umfassende Alkylengruppe darstellt und Z ein Wasserstoffatom ist, während Y wie voranstehend definiert ist.

**8.** Verwendung gemäß Anspruch 7 wobei die sich wiederholende Siloxanstruktureinheit der allgemeinen Formel (II) entspricht, worin $X_1$ eine Ethylengruppe ist, $X_2$ eine Propylengruppe ist und Z ein Wasserstoffatom ist, während Y wie voranstehend definiert ist.

**9.** Verwendung gemäß Anspruch 8, wobei die sich wiederholende Siloxanstruktureinheit der nachstehenden Formel (IV) entspricht,:

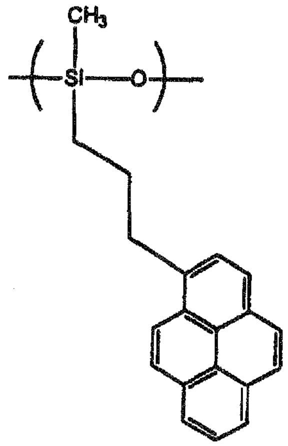

(IV)

**10.** Verwendung gemäß einem der vorangehenden Ansprüche, wobei das fluoreszierende Polysiloxan ein Homopolymer ist.

**11.** Verwendung gemäß den Ansprüchen 6 und 10, wobei das fluoreszierende Polysiloxan ein Homopolymer mit einer sich wiederholenden Siloxanstruktureinheit der Formel (III) ist.

**12.** Verwendung gemäß den Ansprüchen 9 und 10, wobei das fluoreszierende Polysiloxan ein Homopolymer mit einer sich wiederholenden Siloxanstruktureinheit der Formel (IV) ist.

**13.** Verwendung gemäß einem der Ansprüche 1 bis 9, wobei das fluoreszierende Polysiloxan ein Copolymer ist, das das sich aus einer ersten, sich wiederholenden Siloxanstruktureinheit, die einer der allgemeinen Formeln (I) und (II) entspricht, worin $W_1$, $W_2$, V, p, Y, $X_1$, $X_2$ und Z wie voranstehend definiert sind, und einer zweiten, sich wiederholenden Siloxanstruktureinheit zusammensetzt, die der nachstehenden Formel (V) entspricht:

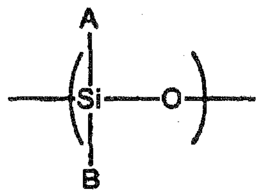

(V)

worin A und B, die gleich oder verschieden sein können, ein Wasserstoffatom oder eine gesättigte oder ungesättigte Kohlenwasserstoffgruppe darstellen, die 1 bis 50 Kohlenstoffatome umfasst und gegebenenfalls ein oder mehrere Heteroatome und/oder eine oder mehrere, wenigstens ein Heteroatom umfassende chemische Funktionen aufweist.

**14.** Verwendung gemäß Anspruch 13, wobei die zweite, sich wiederholende Siloxanstruktureinheit eine Dimethylsiloxanstruktureinheit ist.

**15.** Verwendung gemäß den Ansprüchen 6 und 14, wobei das fluoreszierende Polysiloxan ein Copolymer ist, das sich aus einer ersten, der Formel (IV) entsprechenden, sich wiederholenden Siloxanstruktureinheit und einer zweiten, sich wiederholenden Dimethylsiloxanstruktureinheit zusammensetzt.

**16.** Verwendung gemäß einem der Ansprüche 13 bis 15, wobei das Copolymer statistisch, alternierend oder blockförmig ist.

**17.** Verwendung gemäß einem der vorangehenden Ansprüche, wobei das fluoreszierende Polysiloxan in Form eines dünnen, eine oder beide Seiten eines Substrats bedeckenden Films vorliegt.

**18.** Verwendung gemäß Anspruch 17, wobei der dünne Film in der Dicke 10 Ångström bis 100 Mikron misst.

**19.** Verwendung gemäß einem der vorangehenden Ansprüche, wobei die Sonde eine optische Fluoreszenzsonde ist.

**20.** Verwendung gemäß einem der Ansprüche 1 bis 19, wobei die Sonde eine gravimetrische Sonde, vorzugsweise eine Quarzmikrowaage ist.

**21.** Verwendung gemäß einem der Ansprüche 1 bis 18, wobei die Sonde eine Multisonde ist, die eine oder mehrere optische Fluoreszenzsonden und/oder eine oder mehrere gravimetrische Sonden umfasst und wenigstens eine dieser Sonden wenigstens ein wie voranstehend definiertes, fluoreszierendes Polysiloxan als empfindliches Material umfasst.

**22.** Verwendung gemäß einem der vorangehenden Ansprüche, wobei die Nitroverbindung aus nitroaromatischen Verbindungen, Nitraminen, Nitrosaminen und Salpetersäureestern ausgewählt ist.

**23.** Verwendung gemäß Anspruch 22, wobei die Nitroverbindung aus Nitrobenzol, Dinitrobenzol, Trinitrobenzol, Nitrotoluol, Dinitrotoluol, Trinitrotoluol, Dinitrofluorbenzol, Dinitrotrifluormethoxybenzol, Aminodinitrotoluol, Dinitrotrifluormethylbenzol, Chlordinitrotrifluormethylbenzol, Nexanitrostilben, Trinitrophenol, Cyclotetramethylentetranitramin, Cyclotrimethylentrinitramin, Trinitrophenylmethylnitramin, Nitrosodimethylamin, Pentrit, Ethylenglykoldinitrat, Diethylenglykoldinitrat, Nitroglycerin oder Nitroguanidin ausgewählt ist.

**24.** Verwendung gemäß einem der vorangehenden Ansprüche zum Nachweis von Sprengstoffen.

**25.** Fluoreszierendes Polysiloxan, das eine der nachstehenden allgemeinen Formel (I) entsprechende, sich wiederholende Struktureinheit umfasst:

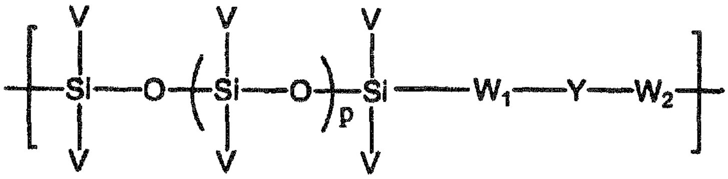

(I)

worin:

$W_1$ und $W_2$, die gleich oder verschieden sein können, eine Kohlenwasserstoffgruppe darstellen, die 2 bis 50 Kohlenstoffatome umfasst und gegebenenfalls ein oder mehrere Heteroatome und/oder eine oder mehrere chemische Funktionen aufweist, die wenigstens ein Heteroatom umfassen;
V ein Wasserstoffatom oder eine Kohlenwasserstoffgruppe darstellt, die 1 bis 50 Kohlenstoffatome umfasst und gegebenenfalls ein oder mehrere Heteroatome und/oder eine oder mehrere chemische Funktionen aufweist, die wenigstens ein Heteroatom umfassen; p eine ganze Zahl von 0 bis 10000 ist; und
Y eine fluorophore Gruppe darstellt.

**26.** Fluoreszierendes Polysiloxan gemäß Anspruch 25, das eine der allgemeinen Formel (ii) entsprechende, sich wiederholende Siloxanstruktureinheit umfasst, worin Y eine fluorophore Gruppe darstellt, die aus einer Naphthacenyl-, Naphthalinyl-, Acenaphthyl-, Dansyl-, Xanthanyl-, Thioxanthanyl-, Anthrachinyl-, Acridinyl-, Rhodaminyl-, Fluorenyl-, Fluoranthrenyl-, Pentacenyl-, Tetrahydrochrysenyl-, Carbazolyl-, Pyridyl-, Imidazolyl-, Phenothiazolyl-, Toluidinyl-, 1 H-Benzimidazo[2,1-a]benz[de]isochinolinyl-, 4-(2'-Phenyl)vinylphenyl-, Anthracenyl-, Pyrenyl-, Perylenyl-, Benzoperylenyl-, Benzo[k]fluoranthenyl, Benzothiazolyl, Benzimidazolyl-, Oligo(phenylenvinylidenyl-), Oligo(phenylenethinylenyl-), Fluoresceinyl-, Cumarinyl-, Pyridyloxazolyl-, Benzoxazolyl-, Benzoxadiazolyl- und 5,5'-Bis(4-aminophenyl)-2,2'-bifurylgruppe und deren funktionellen Derivaten ausgewählt ist.

**27.** Fluoreszierendes Polysiloxan gemäß Anspruch 25 oder Anspruch 26, das eine der allgemeinen Formel (I) entsprechende, sich wiederholende Siloxanstruktureinheit umfasst, worin Y eine Pyrenylgruppe ist.

**28.** Fluoreszierendes Polysiloxan gemäß einem der Ansprüche 25 bis 27, das eine der allgemeinen Formel (I) entsprechende, sich wiederholende Struktureinheit umfasst, worin $W_1$ und $W_2$ unabhängig voneinander eine 2 bis 50 Kohlenstoffatomen umfassende Kohlenwasserstoffgruppe darstellen und V eine 1 bis 10 Kohlenstoffatome umfassende Alkylgruppe darstellt, während p und Y wie voranstehend definiert sind.

**29.** Fluoreszierendes Polysiloxan gemäß Anspruch 28, das eine der allgemeinen Formel (I) entsprechende, sich wiederholende Siloxanstruktureinheit umfasst, worin $W_1$ und $W_2$ alle beide eine Propylengruppe sind, V eine Methylgruppe ist, p 1 ist und Y wie voranstehend definiert ist.

**30.** Fluoreszierendes Polysiloxan gemäß Anspruch 29, das eine der nachstehenden Formel (III) entsprechende, sich wiederholende Siloxanstruktureinheit umfasst:

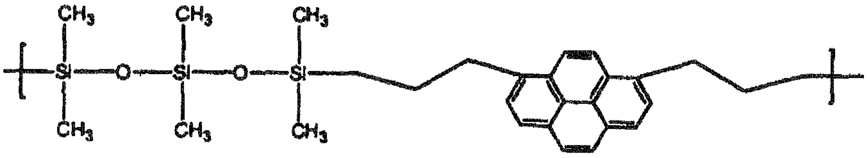

(III)

**31.** Fluoreszierendes Polysiloxan gemäß einem der Ansprüche 25 bis 30, das ein Homopolymer ist.

**32.** Fluoreszierendes Polysiloxan gemäß Anspruch 30 oder Anspruch 31, das ein Homopolymer mit einer sich wiederholenden Siloxanstruktureinheit der Formel (III) ist.

**33.** Chemische Sonde, die wenigstens ein wie in einem der Ansprüche 25 bis 32 definiertes fluoreszierendes Polysiloxan

als empfindliches Material umfasst.

**34.** Chemische Sonde gemäß Anspruch 33, bei der das fluoreszierende Polysiloxan in Form eines dünnen, eine oder beide Seiten eines Substrats bedeckenden Films vorliegt.

**35.** Chemische Sonde gemäß Anspruch 34, bei der der dünne Film in der Dicke 10 Ångström bis 100 Mikron misst.

**36.** Chemische Sonde gemäß einem der Ansprüche 33 bis 35, bei der die Sonde eine optische Fluoreszenzsonde ist.

**37.** Chemische Sonde gemäß einem der Ansprüche 33 bis 35, bei der die Sonde eine gravimetrische Sonde, vorzugsweise eine Quarzmikrowaage ist.

**38.** Chemische Sonde gemäß einem der Ansprüche 33 bis 35, bei der die Sonde eine Multisonde ist, die eine oder mehrere optische Fluoreszenzsonden und/oder eine oder mehrere gravimetrische Sonden umfasst und wenigstens eine dieser Sonden wenigstens ein wie voranstehend definiertes, fluoreszierendes Polysiloxan umfasst.

**39.** Verfahren zur Herstellung eines fluoreszierenden Polysiloxans, das sich aus einer sich wiederholenden, der nachstehenden allgemeinen Formel (I) entsprechenden Struktureinheit zusammensetzt:

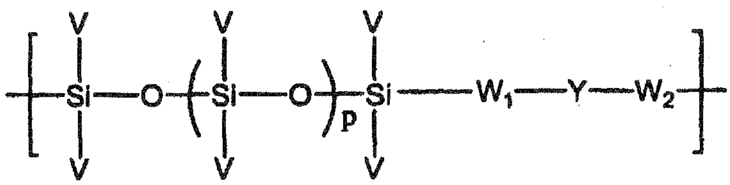

(I)

worin:

$W_1$ und $W_2$, die gleich oder verschieden sein können, eine Kohlenwasserstoffgruppe darstellen, die 2 bis 50 Kohlenstoffatome umfasst und gegebenenfalls ein oder mehrere Heteroatome und/oder eine oder mehrere chemische Funktionen aufweist, die wenigstens ein Heteroatom umfassen;
V ein Wasserstoffatom oder eine Kohlenwasserstoffgruppe darstellt, die 1 bis 50 Kohlenstoffatome umfasst und gegebenenfalls ein oder mehrere Heteroatome und/oder eine oder mehrere chemische Funktionen aufweist, die wenigstens ein Heteroatom umfassen; p eine ganze Zahl von 0 bis 10000 ist; und
Y eine fluorophore Gruppe darstellt,

wobei das Verfahren die Polykondensation einer Verbindung der nachstehenden Formel (VI):

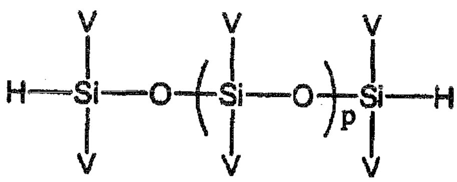

(VI)

worin V und p dieselben Bedeutungen wie in der allgemeinen Formel (I) aufweisen, mit einer Verbindung der nachstehenden Formel (VII) umfasst:

$$P_1\text{——}Y\text{——}P_2 \qquad \text{(VII)}$$

worin:

Y dieselbe Bedeutung wie in der allgemeinen Formel (I) aufweist und
$P_1$ und $P_2$, die gleich oder verschieden sein können, eine Kohlenwasserstoffgruppe darstellen, die 2 bis 50 Kohlenstoffatome umfasst und die eine Endgruppe $-CH=CH_2$ aufweist.

**40.** Verfahren zur Herstellung eines fluoreszierenden Polysiloxans, das sich aus mehreren verschiedenen, sich wiederholenden Siloxanstruktureinheiten zusammensetzt, wovon wenigstens eine der nachstehenden allgemeinen Formel (I) entspricht:

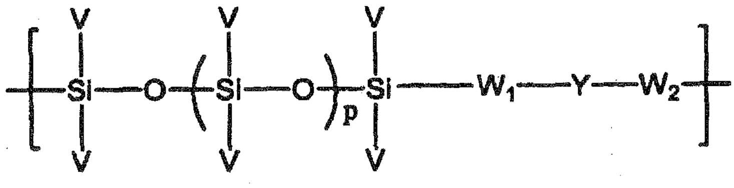

(I)

worin:

$W_1$ und $W_2$, die gleich oder verschieden sein können, eine Kohlenwasserstoffgruppe darstellen, die 2 bis 50 Kohlenstoffatome umfasst und gegebenenfalls ein oder mehrere Heteroatome und/oder eine oder mehrere chemische Funktionen aufweist, die wenigstens ein Heteroatom umfassen;
V ein Wasserstoffatom oder eine Kohlenwasserstoffgruppe darstellt, die 1 bis 50 Kohlenstoffatome umfasst und gegebenenfalls ein oder mehrere Heteroatome und/oder eine oder mehrere chemische Funktionen aufweist, die wenigstens ein Heteroatom umfassen; p eine ganze Zahl von 0 bis 10000 ist; und
Y eine fluorophore Gruppe darstellt,

wobei das Verfahren die Polykondensation eines Monomeren der nachstehenden Formel (VIII):

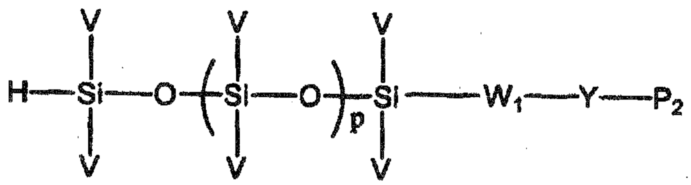

(VIII)

worin:

$W_1$, V, Y und p dieselben Bedeutungen wie in der allgemeinen Formel (I) aufweisen, und
$P_2$ eine Kohlenwasserstoffgruppe darstellt, die 2 bis 50 Kohlenstoffatome umfasst und eine Endgruppe $-CH=CH_2$ aufweist,

oder eines Oligomeren, das sich aus der Kondensation mehrerer Monomeren der Formel (VIII) ergibt, mit einem weiteren monomeren Siloxan oder einem weiteren, aus diesem letzten Monomer gebildeten Oligomer umfasst.

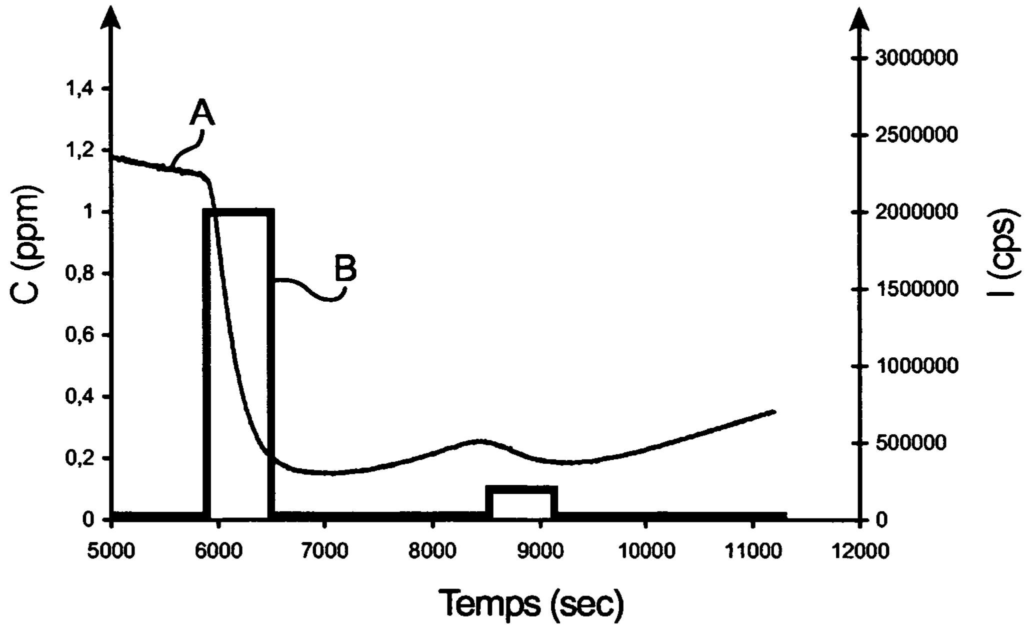
1,4
1,2
1
0,8
0,6
0,4
0,2
0
C (ppm)
A
B
3000000
2500000
2000000
1500000
1000000
500000
0
I (cps)
5000
6000
7000
8000
9000
10000
11000
12000
Temps (sec)

FIG.1

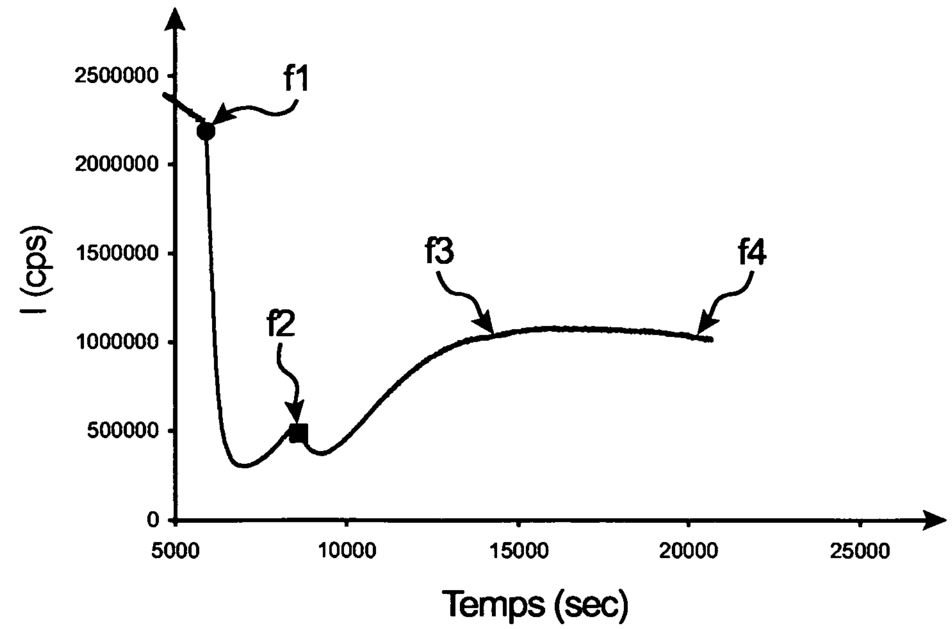
2500000
2000000
1500000
1000000
500000
0
I (cps)
f1
f2
f3
f4
5000
10000
15000
20000
25000
Temps (sec)

FIG.2

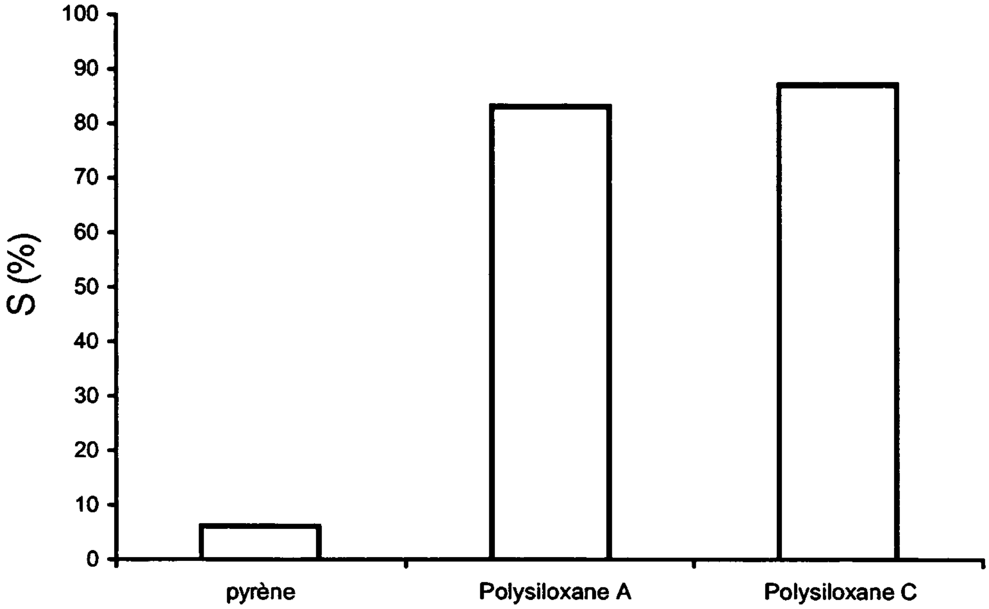
100
90
80
70
60
50
40
30
20
10
0
S (%)
pyrène
Polysiloxane A
Polysiloxane C

FIG.3

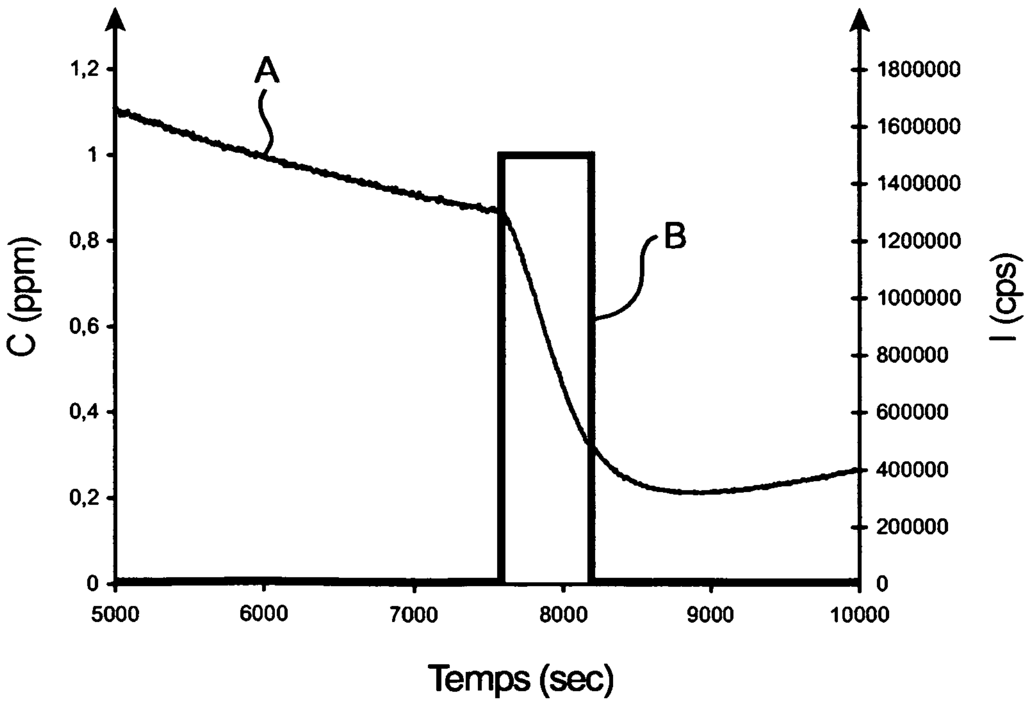
1,2
1
0,8
0,6
0,4
0,2
0
C (ppm)
A
B
1800000
1600000
1400000
1200000
1000000
800000
600000
400000
200000
0
I (cps)
5000
6000
7000
8000
9000
10000
Temps (sec)

FIG.4

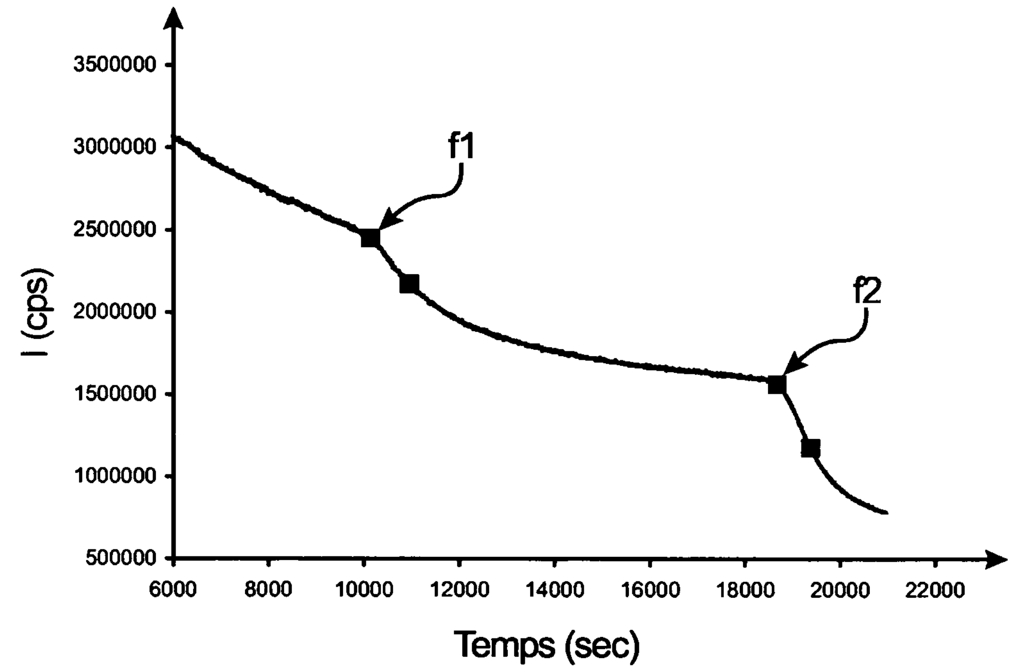
3500000
3000000
2500000
2000000
1500000
1000000
500000
I (cps)
f1
f2
6000
8000
10000
12000
14000
16000
18000
20000
22000
Temps (sec)

FIG.5

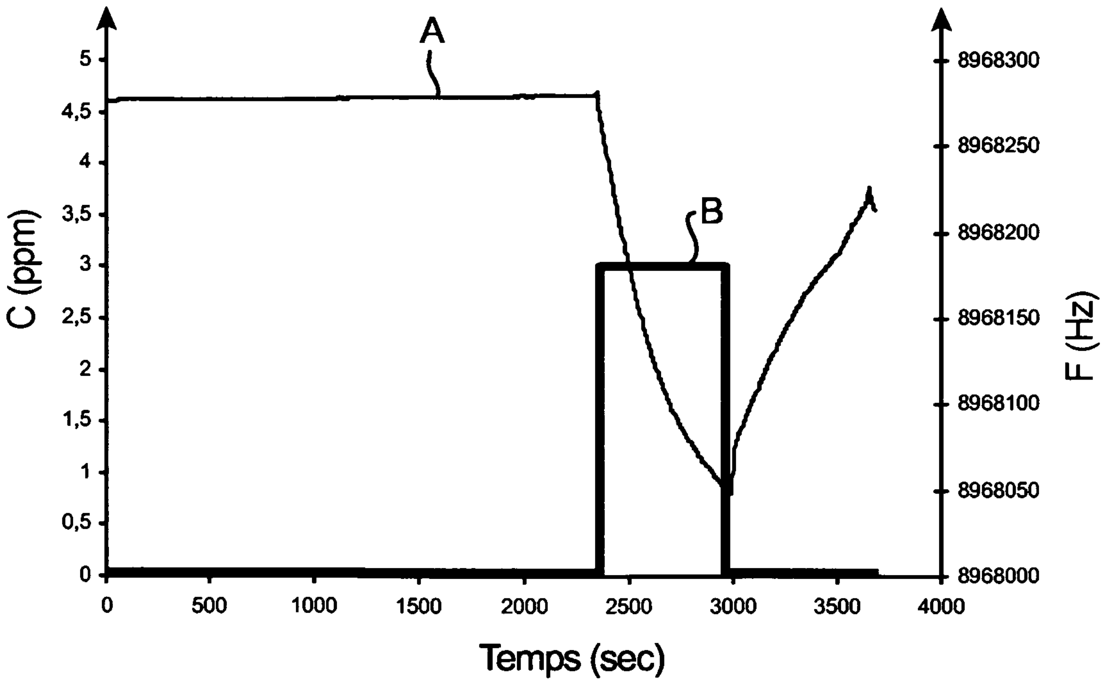
A
B
5
4,5
4
3,5
3
2,5
2
1,5
1
0,5
0
C (ppm)
8968300
8968250
8968200
8968150
8968100
8968050
8968000
F (Hz)
0
500
1000
1500
2000
2500
3000
3500
4000
Temps (sec)

FIG.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9822795 A


### Littérature non-brevet citée dans la description


- Molecular Fluorescence : Principles and Applications. WILEY VCH, 2002
- **J.A.O. SANCHEZ-PEDRONO et al.** *Anal. Chem. Acta,* 1986, vol. 182, 285
- **TAKUWA et al.** *J. Chem. Soc. Perkin Trans,* 1998, vol. 1, 1309
- **MCGILL et al.** *Sensors and Actuators,* 2000, vol. B65, 5-9
- **B. VALEUR.** Molecular Fluorescence : Principles and Applications. WILEY VCH, 2002